(19) Europäisches Patentamt
European Patent Office
Office européen des brevets

(11) **EP 3 159 399 B1**

(12) **EUROPÄISCHE PATENTSCHRIFT**

(45) Veröffentlichungstag und Bekanntmachung des Hinweises auf die Patenterteilung:
**16.11.2022 Patentblatt 2022/46**

(21) Anmeldenummer: **16193758.6**

(22) Anmeldetag: **20.09.2007**

(51) Internationale Patentklassifikation (IPC):
***C12M 1/34*** *(2006.01)*

(52) Gemeinsame Patentklassifikation (CPC):
**C12M 33/04; C12M 41/06; C12M 41/48**

(54) **VERFAHREN UND VORRICHTUNG ZUR AUTOMATISIERTEN ENTNAHME VON ZELLEN UND/ODER ZELLKOLONIEN**

METHOD AND DEVICE FOR THE AUTOMATIC REMOVAL OF CELLS AND/OR CELL COLONIES

PROCÉDÉ ET DISPOSITIF DE PRÉLÈVEMENT AUTOMATISÉ DE CELLES ET/OU DE COLONIES DE CELLULES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **22.09.2006 DE 102006045262**

(43) Veröffentlichungstag der Anmeldung:
**26.04.2017 Patentblatt 2017/17**

(62) Dokumentnummer(n) der früheren Anmeldung(en) nach Art. 76 EPÜ:
**07820393.2 / 2 064 310**

(73) Patentinhaber: **ALS Automated Lab Solutions GmbH**
**07745 Jena (DE)**

(72) Erfinder:
• **Backhaus, Rafael**
  **50733 Köln (DE)**
• **Eberhardt, Jens**
  **07546 Gera (DE)**
• **Meinhard, Marco**
  **04626 Schmölln (DE)**
• **Bornmann, Gerd**
  **99427 Weimar (DE)**

(74) Vertreter: **Riechelmann & Carlsohn Patentanwälte PartG mbB**
**Wiener Straße 91**
**01219 Dresden (DE)**

(56) Entgegenhaltungen:
EP-A- 1 502 649      WO-A-00/13609
WO-A-02/19594       WO-A-91/01365
WO-A1-03/048705     DE-A1-102004 046 740
DE-C2- 19 742 163   US-A- 6 122 396

## Beschreibung

Die Erfindung betrifft eine Vorrichtung zur automatisierten Entnahme von Zellen und/oder Zellkolonien.

Im Rahmen der biologischen bzw. medizinischen Forschung sind Arbeiten zur Pflege, zum Screening und zur Selektion von Zellen und/oder Zellkolonien in Zellkulturen anhand unterschiedlichster Kriterien vorzunehmen. Dabei müssen die Zellen und/oder Zellkolonien gezielt ausgewählt und in andere Probengefäße bzw. Zellkulturen verbracht werden.

Der stetig wachsende Markt der Biotechnologie und medizinischen Forschung ist derzeit geprägt durch den Wandel von weitgehender manueller Zellkurselektion und -pflege hin zu teil- und vollautomatisierten Systemen, welche anhand spezifischer Kriterien Zellen selektieren und separieren.

Auf Grund der in diesem Bereich sehr unterschiedlichen Zelltypen mit stark variierenden Eigenschaften und Ernteverhalten entstehen weitgehend spezielle, auf den zu bearbeitenden Zelltyp angepasste Lösungen.

Da im Bereich der universitären wie auch kommerziellen Forschung in diesem Bereich häufige Wechsel der verwendeten Zelllinien auf Grund wechselnder Forschungsaufgaben stattfinden, ist die Anschaffung dieser teueren und nur speziell einsetzbaren Vorrichtungen für kleinere Forschungsteams wirtschaftlich uninteressant.

Die Praktische Umsetzung erfolgte bisher durch eine Aufnahme unterschiedlicher handelsüblicher Kunststofftips zum Zwecke der Entnahme von Partikeln aus Medien, eine Aufnahme spezieller Kunststofftips mit vergrößerter Öffnung zur Aufnahme von Partikeln aus zähflüssigen Medien, eine Aufnahme metallischer Kapillaren zur Aufnahme stark adhärenter Partikel unterschiedlicher Größe durch Scrapen oder eine Aufnahme von in Adaptern eingebrachten Glaskapillaren zur Separation kleiner, nichtadhärenter oder wenig adhärenter Partikel.

Für die Separation und Kulturpflege stark adhärenter Zellen und Kolonien sind relativ universell verwendbare Geräte zur Automatisierung dieser Arbeiten nicht verfügbar.

Ein Hauptproblem stellt die starke Adhärenz der Zellen am Probengefäß dar. Bekannte Verfahren, wie z.B. das in DE 197 42 163 C2 geschilderte gezielte Klonieren mittels Enzymen zur Lösung der Bindung der Zellmembran vom Probengefäß, scheitern durch eine zu lange Einwirkzeit des Enzyms bis zur Einstellung des gewünschten Effektes und dem durch die Schädigung der Zellmembran auf Grund des Einsatzes das Enzyms einsetzende Absterben von Zellen und starke Verminderung der Vitalität. Somit wird die Automatisierung der gezielten Selektion solcher Zellen in bekannter Weise nicht möglich.

Vor allem im Bereich der Stammzellforschung treten diese Probleme auf.

Die praktische Umsetzung erfolgte bisher durch Scrapekanülen aus Edelstahl mit unterschiedlichen Durchmessern, Scrapekanülen aus Glas oder Metall, welche in einen konischen Adapter eingeklebt werden, oder Scrapekanülen aus Keramik für kleinere Durchmesser.

Auf Grund der meist hohen Anforderungen an Sterilität zum Schutz der Proben vor Verunreinigungen müssen die verwendeten Kanülen in aller Regel nach jedem Vereinzelungszyklus gewechselt werden. Dies erfolgt bei hohem Durchsatz entsprechend häufig und ist manuell nicht zumutbar.

Zur halbautomatischen Verarbeitung, insbesondere zur Vereinzelung, der Isolierung und Behandlung von Zellklonen und Einzelzellen sind bereits eine Reihe von mechanischen Vorrichtungen und Robotsystemen bekannt.

Zum Beispiel offenbart die DE 10 2004 027 661 A1 eine Antriebsanordnung für ein Robotsystem zur Isolierung und Behandlung von Zellklonen und Einzelzellen. Die Antriebsanordnung umfasst motorisch betriebene Schlitten, die in zwei unterschiedlichen Achsrichtungen verfahren werden können und dazu dienen, ein Vereinzelungswerkzeug zu den Proben zu bewegen.

Die DE 10 2004 046 740 A1 beschreibt einen Werkzeugkopf zur automatischen Isolierung und Behandlung von Zellklonen. Dieser Werkzeugkopf dient zur Aufnahme einer Pipette, die wiederum eine Klonierglocke aufnimmt. Durch eine gestaffelte Anordnung von Blattfedern ist ein definierter Kraftschluss zwischen der Pipettenspitze und der Klonierglocke möglich.

In der DE 197 42 163 A1 ist eine Klonierglocke beschrieben, die die Zelle und/oder Zellkolonie bei der Entnahme umschließt und in der ein Spülvorgang erfolgt, mit Hilfe dessen die Zelle und/oder Zellkolonie vom Gefäß gelöst wird. Die durch das Spülen gelöste Zelle und/oder Zellkolonie wird anschließend durch die Klonierglocke hindurch abgesaugt.

Ein automatisiertes Verfahren zum Aufnehmen von Tierzellenkolonien ist zum Beispiel in der EP 1 502 649 A1 offenbart. Die Zellkultur wird mit einer optischen Einrichtung abgescannt und es werden Bilddaten der Zellkultur erzeugt. Diese Bilddaten werden an eine Bildverarbeitungseinheit übermittelt. In der Bildverarbeitungseinheit wird unter Verwendung der Bilddaten eine Formerkennung ausgeführt. Dabei werden Positionsdaten der Zelle und/oder der Zellkolonie erfasst. Die so ermittelten Positionsdaten werden in einer Positionsdatenbank gespeichert. Anschließend werden die Positionsdaten aus der Positionsdatenbank an eine Ernteeinheit übergeben. Die Ernteeinheit wird an einen räumlichen Ort der auf dem Träger befestigten Zellkultur gemäß den vorher ermittelten Positionsdaten bewegt. Es erfolgt dann ein Aufnehmen der Zelle und/oder Zellkolonie am räumlichen Ort der Zellkultur durch die Ernteeinheit. Eine hohle Nadel in einem Aufnahmekopf nimmt die Zellkolonien durch Ansaugen aus der Zellkultur auf. Die Ernteeinheit transportiert die aufgenommene Zelle und/oder Zellkolonie an einen Bestimmungsort und setzt dort die Zelle und/oder Zellkolonie ab.

In der EP 1 754 537 A1 ist ein Verfahren zum Auswählen und Aufnehmen von Tierzellenkolonien beschrieben, bei dem die Zellkolonien mit einem markierten Protein in Kontakt gebracht werden, so dass die Helligkeit der Zellkolonien dem Gehalt an einem gesuchten Protein entspricht. Die Zellkolonien werden dann anhand der Helligkeit ausgewählt.

Bei den bekannten Verfahren werden jedoch nur die Positionen von Zellkolonien bzw. Zellkolonien mit bestimmten Eigenschaften erkannt.

Aus der bereits genannten EP 1 502 649 A1 ist außerdem eine Vorrichtung zur Aufnahme von Tierzellkolonien bekannt. Die Vorrichtung weist einen Aufnahmekopf auf, der eine Anordnung von Hohlnadeln trägt.

Es besteht daher die Aufgabe, aus den erkannten Zellen und/oder Zellkolonien Zellen und/oder Zellkolonien mit speziellen Eigenschaften auszuwählen.

Die Aufgabe wird mit einer Vorrichtung zur automatisierten Entnahme von Zellen und/oder Zellkolonien aus einer Zellkultur mit den Merkmalen des Anspruchs 1 gelöst. Die Unteransprüche enthalten zweckmäßige bzw. vorteilhafte Ausführungsformen und Merkmale der Vorrichtung.

Die erfindungsgemäße Vorrichtung weist die Merkmale von Anspruch 1 auf. Die erfindungsgemäße Vorrichtung ist zur Ausführung des nachstehend beschriebenen Verfahrens geeignet.

Das Verfahren umfasst eine automatisierte Entnahme von Zellen und/oder Zellkolonien aus einer Zellkultur unter Ausführen eines ersten Detektionsschritts zum Auswählen von Zellen und/oder Zellkolonien anhand von physischen und/oder physikalischen Parametern und Erfassen von Positionsdaten und dem Speichern der erfassten Positionsdaten der ausgewählten Zellen und/oder Zellkolonien in einer Positionsdatenbank.

Danach werden mindestens ein zweiter Detektionsschritt zur Erkennung mindestens eines weiteren Parameters der Zellen und/oder Zellkolonien ausgeführt, Vergleichsdaten aus den Daten des zweiten Detektionsschritts und den Daten des ersten Detektionsschritts erstellt, Zellen und/oder Zellkolonien anhand der Vergleichsdaten ausgewählt und die Positionsdaten aus der Positionsdatenbank an eine Ernteeinheit übergeben. Zum Ausführen des ersten Detektionsschritts während der Bildverarbeitung werden physische und/oder physikalische Parameter, insbesondere Flächenausdehnungen, Größen und/oder Umrisse, und/oder spektrale Parameter, insbesondere Helligkeiten und/oder Fluoreszenzintensitäten erfasst. Derartige Parameter können standardmäßig zur Identifikation der gesuchten Zellen und/oder Zellkolonien herangezogen werden und lassen sich in relativ einfacher Weise aus den Bildpunkten der erfassten Bildinformationen ableiten.

Der zweite Detektionsschritt wird zur weiteren Untersuchung der Zelle und/oder Zellkolonie nach dem Abscannen und der Formerkennung eingeführt, um die bereits erkannten Zellen und/oder Zellkolonien hinsichtlich weiterer Parameter zu untersuchen. Bei dieser Analyse werden nicht mehr die gesamte Ausgangsplatte gescannt, sondern nur noch die Bereiche, in denen bei der Formerkennung interessantes Material gefunden wurde. Dies optimiert die Ausführungszeit und im Falle einer Fluoreszenzanalyse auch die Beleuchtungsdauer der Zelle und/oder Zellkolonie mit Fluoreszenzlicht, die möglichst kurz sein soll, um ein Ausbleichen der Probe zu verhindern.

Grundgedanke des Verfahrens ist es also, in einem automatisierten, mehrschrittigen Prozess Zellen und/oder Zellkolonien mit mehreren bestimmten Eigenschaften innerhalb einer Zellkultur aufzufinden, nach den gewünschten Eigenschaften auszuwählen, aufzunehmen und weiter zu verarbeiten.

Bei einer zweckmäßigen Ausführungsform ist als ein Träger des Gefäßes der Zellkultur ein xy-Tisch vorgesehen. Dabei erfolgt das Abscannen der Zellkultur und das Anfahren der Ernteeinheit durch eine Bewegung des xy-Tisches. Bei dieser Vorgehensweise führt somit der xy-Tisch schrittweise die Zellkultur unter einer Bilderfassungseinheit entlang einzelner Koordinatenpunkte hindurch, wobei eine Bildserie aufgenommen und verarbeitet wird. Das Anfahren der identifizierten Orte auf der Zellkultur durch die Ernteeinheit erfolgt dann in entsprechender Weise so, dass der xy-Tisch die Zellkultur bezüglich der Ernteeinheit so verschiebt, dass sich diese über der entsprechenden Koordinatenposition befindet. Dadurch werden aufwändige Verschiebe- und Stellmechaniken für Bilderfassung und Ernteeinheit eingespart, und es ist nur eine Verstelleinrichtung für beide Verfahrensabschnitte notwendig.

Vorzugsweise werden beim Abscannen Teilbilder der Zellkultur aufgenommen und der erste und/oder zweite Detektionsschritt anhand der Teilbilder durchgeführt. Dies stellt einen großen Vorteil hinsichtlich des benötigten Bildspeichers und der Bildqualität bei der Detektion dar. Die Bilderkennung kann außerdem an den softwareseitig unveränderten Bilddaten und damit an der physikalisch bestmöglichen Auflösung und Bildtreue erfolgen. Es kann mit der vollen Auflösung bei der eingestellten Vergrößerung gearbeitet werden, während bei der Analyse am zusammengesetzten Bild, die weiter unten beschrieben ist, meist kleiner gezoomt wird, um die Bilddatenmenge im Rahmen zu halten.

Alternativ dazu wird bei dem Verfahrensschritt des Abscannens zweckmäßigerweise eine Gesamtheit aus die Zellkultur überdeckenden Teilbildern aufgenommen. Dabei werden die Bilddaten der Teilbilder in der Bildverarbeitungseinheit zu Bilddaten eines Übersichtsbildes der Zellkultur vereinigt. Eine derartige Vorgehensweise ist zweckmäßig, weil unter den Bedingungen einer mikroskopischen Abbildung immer nur ein Ausschnitt der Zellkultur erfasst werden kann, andererseits aber die absolute Lage einer Zelle bzw. Zellkolonie im Bereich der Zellkultur für die spätere Entnahme bekannt sein muss. Das Übersichtsbild der Zellkultur besteht unter diesen Bedingungen zwangsläufig aus einem Mosaik von Teilbildern.

Bei dem Erfassen der Positionsdaten erfolgt ein Ermitteln des Formenschwerpunktes erkannter Formen. Dabei werden die Koordinaten des Formenschwerpunkts als die Positionsdaten der erkannten Form in der Positionsdatenbank gespeichert. Diese zweckmäßige Vorgehensweise definiert für eine erkannte Form bzw. Kontur deren Lage mit einem verhältnismäßig geringen Datenumfang. Dabei werden die zu einer Form gehörenden Bildpunkte zu einem Referenzbildpunkt vereinigt, der die Lage der ermittelten Zelle bzw. Zellkolonie angibt.

Die Formerkennung und das Erfassen der Positionsdaten schließt zweckmäßigerweise ein Ermitteln von Abständen zwischen den erkannten Formen ein. Durch diese Ausgestaltung des Verfahrens kann unter anderem festgelegt werden, welche erfasste Zelle bzw. Zellkolonie noch zu einem erkannten Verband gehört oder einzeln zu entnehmen ist. Dabei kann insbesondere die Reichweite berücksichtigt werden, mit der bei einem gegebenen Erntewerkzeug Zellen bzw. Zellkolonien in der Umgebung der ermittelten Positionsdaten mit einem einzigen Zugriff entnommen werden können. Weiter entfernt gelegene Zellen bzw. Zellkolonien müssen dann separat angefahren werden.

Zweckmäßigerweise erfolgt wahrend des Abscannens, der Formerkennung und/oder dem Erfassen der Positionsdaten eine Echtzeitdarstellung der Bilddaten auf einem Monitor. Dadurch kann die gesamte Prozedur fortlaufend überwacht und gegebenenfalls beeinflusst werden.

Vorzugsweise besteht der zweite Detektionsschritt aus beliebig vielen Einzelschritten, in denen verschiedene Parameter erkannt werden, und die Parameter werden bei den Einzelschritten mit unterschiedlichen Belichtungsarten aufgenommen. So können beim zweiten Detektionsschritt viele verschiedene Fluoreszenzkanäle und Anregungswellenlängen gescannt und bewertet werden. Die Software sammelt hierbei lediglich Partikeldaten, die weniger datenintensiv sind als Bilddaten.

In einer vorteilhaften Ausgestaltung des Verfahrens erfolgt das Auswählen der Zellen und/oder Zellkolonien anhand von physischen und/oder physikalischen Parametern mittels einer ersten interaktiven Auswahlliste. Das Auswählen von Zellen und/oder Zellkolonien anhand der Vergleichsdaten erfolgt mittels einer zweiten interaktiven Auswahlliste oder eines interaktiven Scatter-Diagramms. In dem Scatter-Diagramm werden zwei verschiedene Partikelwerte aus der Ergebnisliste gegeneinander abgebildet. Durch die interaktiven Auswahllisten bzw. das interaktive Scatter-Diagramm ist ein automatisiertes Auswählen der Zellen und/oder Zellkolonien möglich.

Vorzugsweise enthält die erste interaktive Auswahlliste mindestens Koordinaten der Formenschwerpunkte und Bilddaten und/oder Daten aus dem ersten Detektionsschritt. Die zweite interaktive Auswahlliste enthält mindestens die Daten aus der ersten Auswahlliste und dem zweiten Detektionsschritt und/oder die Vergleichsdaten. Mit Hilfe der Auswahllisten ist es möglich, manuell die Positionsdatenbank einzusehen und die automatisiert gefundenen Zellen bzw. Zellkolonien zu überprüfen und zu selektieren. Dadurch können Auswahlfehler korrigiert werden. Das Abspeichern dieser Daten ist außerdem im Gegensatz zum Abspeichern von Bilddaten weniger aufwendig.

Das Auswählen der Zellen und/oder Zellkolonien erfolgt vorzugsweise durch logische Filter. Die Zellen und/oder Zellkolonien können dadurch logisch über das Vorhandensein bzw. das Nichtvorhandensein einzelner Fluoreszenz- oder Hellfeldsignale gefiltert werden.

Das Aufnehmen der Zelle und/oder Zellkolonie erfolgt bei einer adhärenten, d.h. auf dem Boden eines Behältnisses haftenden Zellkultur mit folgenden zweckmäßigen Schritten: Als erstes wird ein Tip aufgenommen und mit einem Enzym oder Lösungsmittel befüllt. Im Anschluss daran werden eine Klonierglocke aufgenommen und eine Zelle und/oder Zellkolonie durch die Klonierglocke umschlossen. Das im Tip enthaltene Lösungsmittel oder Enzym wird anschließend von dem Tip in einen Innenraum der Klonierglocke abgegeben. Die Klonierglocke wird gespült, dabei werden die Zelle und/oder die Zellkolonie abgelöst. Die Zelle und/oder die Zellkolonie werden nun abgesaugt.

Bei einer stark adhärenten Zellkultur erfolgt das Aufnehmen einer Zelle und/oder der Zellkolonie mit folgenden zweckmäßigen Schritten:
Als erstes wird die Zelle und/oder Zellkolonie mit einer Spitze einer Kanüle umschlossen. Anschließend wird eine Relativbewegung der Kanülenspitze in der xy-Ebene ausgeführt und die Zelle und/oder Zellkolonie abgekratzt. Die abgekratzte Zelle und/oder Zellkolonie wird anschließend in die Kanüle aufgenommen. Eine derartige Vorgehensweise ist dann empfehlenswert, wenn die Zelle bzw. die Zellkolonie auf deren Unterlage stark haftet und ein Ablösen durch ein Lösungsmittel bzw. ein Enzym das Risiko einer Zerstörung oder Schädigung der Zelle oder Zellkolonie stark erhöhen würde.

In einer alternativen Ausführungsform wird die Relativbewegung der Kanülenspitze und/oder des xy-Tisches mit einem Saug-und/oder Spülvorgang in der Kanülenspitze kombiniert. Dies ist besonders vorteilhaft, wenn Stammzellen aufgenommen werden sollen.

Wird die gewünschte, ermittelte Zelle und/oder Kolonie und/oder Teilbereich einer Kolonie von einem Kanülrohr umschlossen und durch Relativbewegungen des Werkzeuges und/oder des Probengefäßes auf dem Kreuztisch vom Boden des Probengefäßes abgelöst und in die Kanüle aufgenommen, können im Vergleich zu bisherigen Verfahren in wesentlich kürzerer Zeit und wesentlich schonender entsprechende Zellen und/oder Kolonien vereinzelt werden.

Oftmals wird durch die Verwendung dieses Verfahrens erstmals eine Automatisierung dieser Vorgänge für bestimmte Zellarten überhaupt erst möglich.

Ein Hauptanwendungsgebiet dieser neuartigen Technologie zur Separierung stark adhärenter Zellen stellt die Forschung im Bereich tierischer und humaner Stammzellen dar. Auch das gezielte Herauslösen von Bereichen aus festen Zellverbänden (Zellrasen) wird durch diese Methodik möglich.

Auch die gezielte Separation von Teilbereichen einer Zellkolonie (z.B. undifferenzierter Stammzellen, welche von bereits differenzierten Stammzellen umgeben sind) wird dadurch automatisiert möglich.

Das o.g. Scrapeverfahren hat gegenüber herkömmlichen, oszillierenden Verfahren weiterhin den Vorteil, dass das unerwünschte, unkontrollierte Ablösen einzelner Zellen aus dem Kolonieverband durch das Oszillieren, praktisch nicht auftritt. Die Struktur der zu erntenden Kolonie bleibt weitgehend unverändert erhalten.

Zum Aufnehmen einer Zelle und/oder Zellkolonie von einem halbfesten Nährboden, insbesondere Agar oder Methylzellulose, werden zweckmäßigerweise folgende Schritte ausgeführt:

Als erstes wird ein Tip aufgenommen. Der Tip wird anschließend über der Zelle und/oder der Zellkolonie positioniert und die Zelle und/oder die Zellkolonie wird durch den Tip eingeschlossen. Es erfolgt anschließend ein Absaugen der Zelle und/oder Zellkolonie und des Nährbodens in der Umgebung der Zelle und/oder Zellkolonie in den Tip.

Ortsfeste Einzelzellen werden zweckmäßigerweise mit folgenden Schritten aufgenommen:

Es wird in einem ersten Schritt eine Kapillare mit einem Fluid, insbesondere Luft oder einer Flüssigkeit, in einer kalibrierten Menge befüllt. Die Kapillaröffnung wird über der Einzelzelle und/oder Einzelkolonie positioniert. Das Medium in einer Umgebung der Einzelzelle und/oder Einzelkolonie wird in die Kapillare gesaugt, wobei die Einzelzelle bzw. Einzelkolonie mit in die Kapillare aufgenommen wird.

Das Befüllen der Kapillare mit der kalibrierten Menge des Fluids erfolgt bei einer zweckmäßigen Variante unter einer Bilderfassung der Kapillare in Verbindung mit einer Bilddatenauswertung in einer Bildverarbeitungseinheit.

Vorzugsweise wird eine Zellkolonie, deren Größe die verwendbaren Durchmesser des Tips, der Kanüle oder der Kapillare überschreitet, in Teilen nacheinander geerntet. Auf diese Weise können auch relativ große Zellkolonien geerntet werden.

Alternativ werden mit Hilfe des Tips, der Kanüle oder der Kapillare einzelne Teile aus einer Zellkolonie herausgelöst. So können gezielt Bereiche einer Zellkolonie, die bestimmte Eigenschaften aufweisen, getrennt von anderen Bereichen geerntet werden. Ein solches Verfahren bietet sich besonders bei der Ernte von undifferenzierten Stammzellen, welche von differenzierten Stammzellen umgeben sind, an.

In einer weiteren alternativen Ausführungsform werden einzelne Bereiche aus festen Zellverbänden herausgelöst. Auf diese Weise können bestimmte Bereiche mit gewünschten Eigenschaften von den Bereichen mit unerwünschten Eigenschaften getrennt werden.

Vorzugsweise werden die Zellen und/oder Zellkolonien beim Absetzen sortiert in einen Ablagebehälter abgelegt und die Positionsdaten der abgelegten Zelle und/oder Zellkolonie erfasst und verarbeitet. So ist es möglich, geerntete Zellen und/oder Zellkolonien nach Klassen (Dichte, Fluoreszenz, etc.) sortiert in die Ablagebehälter ablegen zu lassen, was für nachgelagerte Prozesse wertvoll sein kann. Außerdem erhält die Bildverarbeitungssoftware nach jedem Erntevorgang die Information, in welche Behälter und an welcher Position in diesem Behälter die Robotersteuerung die Zelle und/oder Zellkolonie abgelegt hat, womit eine vollständige Protokollierung des Gesamtprozesses gewährleistet ist.

Das Verfahren wird vorzugsweise zur Entnahme von Stammzellen, biologischen und/oder chemischen Partikeln oder Festkörpern, insbesondere Beads, verwendet.

Die erfindungsgemäße Vorrichtung zur Entnahme einer Zelle und/oder einer Zellkolonie aus einer Zellkultur weist eine Mikroskopeinheit zur mikroskopischen Abtastung der Zellkultur in Kombination mit einer Bilderfassungseinheit und einer Bildauswerteeinheit zur Positionsdetektierung der Zelle und/oder der Zellkolonie in der Zellkultur, eine Steuer- und Speichereinheit zum Speichern der detektierten Position der Zelle und/oder Zellkolonie und ein Erntemodul mit einem Entnahmewerkzeug zur Entnahme der Zelle und/oder Zellkultur an der detektierten Position der Zelle und/oder Zellkolonie auf.

Durch die Schaffung der erfindungsgemäßen Vorrichtung soll ein Weg aufgezeigt werden, wie mit einer einzigen Vorrichtung eine Vielzahl auch völlig verschiedener Zelltypen bearbeitet werden kann. Hierzu besteht die Möglichkeit, die modulare Vorrichtung mit verschiedenen Werkzeugen und Softwareabläufen auszustatten.

Diese Vorrichtungen mit einer Antriebsanordnung nach DE 10 2004 027 661 und mit den verwendeten zelltypischen Entnahmewerkzeugen im Werkzeugkopf nach DE 10 2004 046 740 sind in der Lage, die im Rahmen der automatisierten Zellernte zur Anwendung kommenden verschiedenen Verfahren zur Vereinzelung unterschiedlichster Einzelzellen und Zellkolonien umzusetzen.

Bei diesen Verfahren werden in aller Regel Kapillaren unterschiedlicher Größen, Formen und Materialen zum Lösen und/oder Aufnehmen der Klone oder Partikel angewendet.

Durch die Realisierung weitgehend einheitlicher Adapter für diese unterschiedlichen Kanülenformen kann die Verwendung weitgehend gleicher Werkzeuge für die unterschiedlichsten Anwendungen ermöglicht werden, was eine hohe Kosteneffizienz und drastische Reduzierung des Entwicklungsaufwandes und der Entwicklungszeit nach sich zieht.

Bei einer Ausführungsform der Vorrichtung besteht das Entnahmewerkzeug aus einem mit einer lösenden oder enzymatischen Flüssigkeit füllbaren Tip, einer an den Tip ankoppelbaren, ausgewählte Zellen und/oder Zellkolonien überdeckenden, mit der innerhalb des Tips enthaltenen Flüssigkeit füllbaren Klonierglocke sowie einer Beschickungs- und Abgabevorrichtung für den Tip und die Klonierglocke.

Bei einer weiteren Ausführungsform ist ein Entnahmewerkzeug vorgesehen, das in Form eines Magazins mit einer Anordnung aus innerhalb des Magazins gelagerten Kanülen unterschiedlichen Durchmessers und einer Koppeleinheit zur selbsttätigen Entnahme der Kanülen aus dem Magazin und deren Integration in einen Wechselkopf ausgebildet ist.

Die Kanülen sind vorzugsweise in unterschiedlichen Größen, Formen und/oder Materialien ausgebildet. Dadurch ist eine Anpassung an die Eigenschaften der zu erntenden Zellen und/oder Zellkolonien möglich.

Diese und zukünftig weitere Verfahren lassen sich durch Ergänzung der Palette an verwendbaren Kanülen realisieren, womit durch geringfügige mechanische Änderungen des Werkzeugkopfes und Anpassungen in der Ablaufsoftware weitergehende Applikationen ohne großen technischen Entwicklungsaufwand eröffnet werden.

Bei einer weiteren Ausführungsform ist ein Entnahmewerkzeug mit einem Saugtip mit einem vergrößerten Querschnitt in dessen Spitzenabschnitt vorgesehen.

Schließlich ist bei einem Entnahmewerkzeug in einer weiteren zweckmäßigen Ausführungsform eine Kapillare, eine Bildaufnahmevorrichtung zum Überwachen einer in der Kapillare enthaltenen Fluidmenge, einer Bildverarbeitungsvorrichtung zum Verarbeiten der Bildinformation der Kapillare und einer Saugvorrichtung zum Absaugen von Zellen und/oder Zellkolonien in die Kapillare vorgesehen.

Die Vorrichtung soll nachfolgend anhand von Ausführungsbeispielen näher erläutert werden. Zur Verdeutlichung dienen die beigefügten Figuren. Es werden für gleiche oder gleich wirkende Teile bzw. Verfahrensschritte die selben Bezugszeichen verwendet. Es zeigen:

Fig. 1 eine Vorrichtung zum Ausführen des Verfahrens in einer beispielhaften Ausführungsform,

Fig. 2 ein beispielhaftes Entnahmewerkzeug aus einem Tip und einer Klonierglocke, Fig. 3 ein beispielhaftes Kanülenmagazin mit einer Reihe von Kanülen und einem Werkzeugkopf mit einem Adapter,

Fig. 4 zwei Ausführungsformen beispielhafter Saugtips mit vergrößerten Querschnitten im Spitzenbereich,

Fig. 5 eine beispielhafte Kapillare mit einer Bildaufnahmevorrichtung zur Kalibrierung,

Fig. 6 einen ersten Verfahrensschritt mit einer Aufnahme von Teilbildern und einem Zusammenfügen der Teilbilder zu einem Übersichtsbild,

Fig. 7 ein beispielhaftes ausgewähltes Teilbild mit Zellen und einer Zellkolonie,

Fig. 8 eine schematische Formerkennung ausgewählter Zellen und Zellkolonien,

Fig. 9 schematische Formschwerpunkte und Positionskoordinaten der erkannten Zellen und Zellkolonien und

Fig. 10 zeigt ein beispielhaftes Flussdiagramm des grundsätzlichen Ablaufs der automatischen Zellernte mit der Vorrichtung.

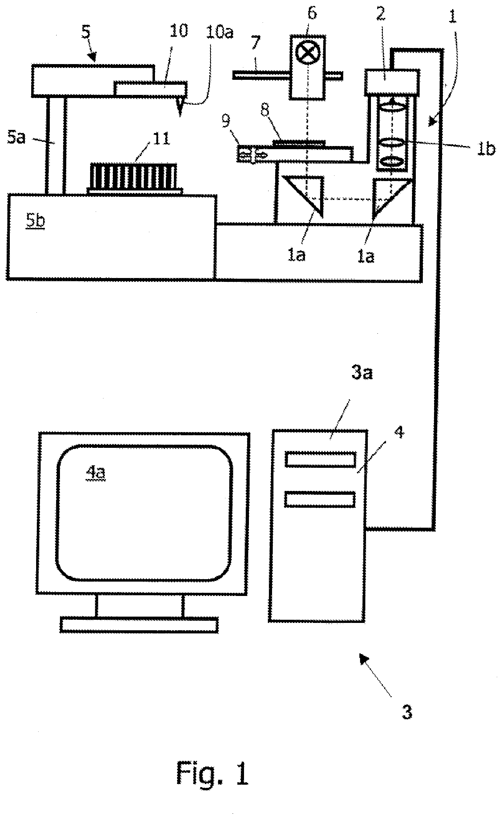

Fig. 1 zeigt eine beispielhafte Ausführungsform einer Vorrichtung zur Entnahme einer Zelle und/oder Zellkultur. Die Vorrichtung enthält eine Mikroskopeinheit 1 mit einer Reihe optischer Komponenten, insbesondere eine Anordnung aus Umlenkprismen l a und ein Linsensystem l b für eine Strahlenführung und mikroskopische Abbildung. Die Mikroskopeinheit 1 ist mit einer Bildaufnahmeeinheit 2, in der Regel einer CCD-Kamera bzw. einem CCD-Feld, gekoppelt. Zur Verarbeitung der aus der Bildaufnahmeeinheit 2 ausgelesenen Bildinformation ist eine Bildauswerteeinheit 3a vorgesehen. Die Bildauswerteeinheit 3a besteht aus einem Personal Computer 3 mit einer darauf ablaufenden Bildverarbeitungssoftware. Weiterhin ist eine Steuer- und Speichereinheit 4 vorgesehen, die in den Personal Computer 3 integriert ist und deren Funktionen durch weitere Softwarekomponenten realisiert ist. Die Steuer- und Speichereinheit 4 weist einen Monitor bzw. ein Display 4a auf.

Die Vorrichtung enthält weiterhin ein Erntemodul 5, das auf einer Verfahrmechanik montiert ist. Die Verfahrmechanik besteht aus einer Hubsäule 5a und einem Verfahrantrieb 5b. Die Hubsäule 5a und der Verfahrantrieb 5b sind für größere Verfahrwege ausgelegt und dienen dem Heranführen des Erntemoduls 5 an eine in einem Probenbehälter befindliche Zellkultur 8, einer Grobeinstellung des Erntemoduls 5 und einem Bewegen eines Entnahmewerkzeugs 10a an die entsprechenden Vereinzelungsstationen der entnommenen Zellen und/oder Zellkolonien.

Die erwähnte Mikroskopeinheit 1 ist als ein Durchlichtmikroskop ausgebildet. Hierzu ist eine Beleuchtung 6 mit einer Reihe von zuschaltbaren Beleuchtungsfiltern 7 vorgesehen. Die Beleuchtung 6 durchstrahlt die in dem Probenbehälter liegende Zellkultur 8. Die Zellkultur 8 ist auf einem Träger in Form eines xy-Tisches 9 befestigt, mit dem die Zellkultur 8 mit einer mikroskopischen Einstellgenauigkeit von einigen Mikrometern sowohl in x-, als auch in y-Richtung unter der optischen Anordnung aus Beleuchtung 6 und darunter befindlichem Umlenkprisma 1a bewegt werden kann.

Dabei werden die Stellkoordinaten des xy-Tisches 9 an die Speicher- und Steuereinheit 4 übertragen bzw. von der Speicher- und Steuereinheit 4 eingestellt.

Die Mikroskopeinheit 1 besteht aus einem handelsüblichen Mikroskopstativ, welches mit einem motorisierten xy-Tisch 9 ausgerüstet ist. Optional kann diese Mikroskopeinheit 1 auch mit einer handelsüblichen Fluoreszenzeinrichtung ausgerüstet werden. Die Fluoreszenzeinrichtung kann bis zu Filtercubes (bestehend aus Anregungsfilter, dichroidem Spiegel und Emissionsfilter) aufnehmen und entweder über einen handelsüblichen Gasbrenner oder über eine mit Glasfaser eingekoppelte externe Beleuchtung 6 beleuchtet werden. Es besteht auch die Möglichkeit, die Emissionsfilter in ein motorisiertes Filterrad vor der Beleuchtung 6 zu setzen, um dann mit entsprechenden Triple- oder Quad-Band-Filter-Cubes in der Fluoreszenzeinrichtung Fluoreszenzen gleichzeitig zu scannen. Außerdem wird die Bildaufnahmeinheit 2 mit CCD-Chip auf der Mikroskopeinheit 1 montiert, durch welche ein Abscannen der Probe möglich ist. Durch den Einsatz von handelsüblichen Phasenkontrastschiebern ist eine physikalisch optimale Phasenkontrastbeleuchtung möglich.

Der handelsübliche PC ist über eine Netzwerkverbindung mit dem Grundgerät verbunden. Auf diesem läuft eine handelsübliche Standardbildverarbeitungssoftware, welche zusammen mit einer eigens programmierten Robotersteuerung sowie eigens entwickelten Modulen für diese Bildverarbeitungssoftware die Ansteuerung des Gerätes und die Analyse der Bilddaten übernimmt.

In Verbindung mit der Bewegung des xy-Tisches 9 wird, wie im folgenden näher erläutert wird, der gesamte Bereich der Zellkultur abgetastet, wobei eine Reihe von mikroskopischen Einzelbildern der Zellkultur durch die Bilderfassungseinheit aufgenommen wird.

Die Bewegung des xy-Tisches dient auch dazu, die Zellkultur für eine Entnahme der aufgefundenen Zellen bzw. Zellkolonien zu positionieren. Hierzu wird das Erntemodul 5 durch die Verfahrmechanik über der Zellkultur 8 positioniert, während der xy-Tisch 9 auf die zuvor ermittelten Positionen der gefundenen Zelten und Zellkolonien eingestellt wird und dem Erntemodul 5 ein Entnehmen der Zellen bzw. Zellkolonien ermöglicht.

Das Entnehmen der Zellen bzw. Zellkolonien aus der im Probenbehälter befindlichen Zellkultur 8 erfordert ein Absenken eines Entnahmewerkzeugs 10a in die Zellkultur 8, ein Aufnehmen der Zellen bzw. Zellkolonie und deren Vereinzeln. Das Entnahmemodul 5 weist hierzu einen Werkzeugkopf 10 auf, der mit einer Absenk- und Saugmechanik ausgestattet ist. An dessen Ende befindet sich das Entnahmewerkzeug 10a. Die aufgenommenen Zellen bzw. Zellkolonien werden in einer Vereinzelungsbatterie 11 abgelegt. Diese besteht aus einer Reihe von Reagenzgläsern bzw. Röhrchen, die durch die Hubsäule und den Verfahrtrieb einzeln angefahren werden können und in die durch den Werkzeugkopf die entnommenen Zellen und Zellkolonien abgesetzt werden können.

Zusätzlich dazu kann die Vereinzelungsbatterie auch in Teilen als ein Magazin zum Bereitstellen von Entnahmewerkzeugen 10a ausgebildet sein, die wahlweise an den Werkzeugkopf 10 angekoppelt werden können, wie im folgenden näher dargestellt wird.

Grundsätzlich werden die hier im Prinzip beschriebenen Funktionen durch die Speicher- und Steuereinheit 4 gesteuert und laufen im wesentlichen vollautomatisch ab. Dem Benutzer stehen jedoch durch die Überwachung der Funktionen am Monitor bzw. Display durch die bekannten Eingabemittel wie Tastatur und Maus und eine entsprechende Nutzeroberfläche bei den innerhalb der Speicher- und Steuereinrichtung ablaufenden Softwarekomponenten eine Reihe von Möglichkeiten zur Funktionsbeeinflussung zur Verfügung.

So ist insbesondere durch einen Zugriff auf die Steuerung der Mikroskopeinrichtung eine Einstellung des Vergrößerungsfaktors und eine Änderung des Auflösungsvermögens der Bildaufnahmeeinrichtung möglich. Weiterhin können durch eine Steuerung der Beleuchtung und der Beleuchtungsfilter Spektralbereiche geändert oder die Mikroskopeinrichtung in einen Fluoreszenz oder Dunkelfeldbetrieb geschaltet werden.

Des weiteren ist eine Adressierung des Erntemoduls 5 möglich, bei der menügesteuert einzelne, durch die Mikroskopeinheit ermittelte Zellen oder Zellkolonien ausgewählt und einem bestimmten Platz in der Vereinzelungsbatterie 11 zugeordnet werden können. Außerdem kann auch eine Betriebsweise des Erntemoduls festgelegt werden, bei der in Abhängigkeit von den selektierten Zellen oder Zellkolonien bestimmte Entnahmewerkzeuge 10a von der konischen Aufnahme 32 des Werkzeugkopfs 10 aufgenommen werden, um die selektierten Zellen auf eine bestimmte Art und Weise zu entnehmen.

Ab diesem Punkt treten nun, entsprechend dem jeweils gewählten Vereinzelungsverfahren unterschiedliche Entnahmewerkzeuge 10a in Aktion, die mit Hilfe unterschiedlicher Kanülen 15, 18 und/oder Kapillaren und unterschiedlichen Abläufen die Partikel vereinzeln und in einen entsprechenden Zielbehälter verbringen. Nachfolgend sind diese Verfahren detailliert beschrieben.

Das Entnahmewerkzeug 10a sitzt am oberen Ende der Antriebsanordnung und kann je nach Applikation frei gewechselt werden. Es können fünf verschiedene Entnahmewerkzeuge 10a für die Applikationen "Adhärente Zellkolonieernte mit Enzym", "Adhärente Zellkolonieernte ohne Enzym durch abkratzen", "Ernte aus Agar", "Ernte aus Methylzellulose" sowie "Ernte von ortsfesten Einzelzellen" verwendet werden. All diesen Entnahmewerkzeugen 10a ist gemeinsam, dass der applikationsspezifische Softwarepart direkt im Entnahmewerkzeug 10a gelagert wird, so dass mit dem Aufstecken des Entnahmewerkzeuges 10a die PC-Software automatisch die richtige Applikation ausführt und die richtigen Verbrauchsmaterialien auf der Vorlagenaufnahme anfährt. Für den bei einigen der Entnahmewerkzeuge 10a als Werkzeugkopf 10 verwendeten Aufbau wird auf die DE 10 2004 046 740 verwiesen. Die Beschreibung der oben genannten einzelnen Applikationen erfolgt nachstehend.

Allen Applikationen ist gemeinsam, dass der Scan- und Analyseprozess gleich abläuft. Erst beim Ernten bzw. Picken der Partikel unterscheidet sich der Prozess je nach Applikation und damit je nach verwendetem Entnahmewerkzeug 10a.

Ernte adhärenter Zellkolonien mittels Enzym:

Dieses Verfahren dient der vollständigen oder teilweisen Ablösung von Zellkolonien, welche am Boden von Probenbehältern anhaften (bei diesen Zelltypen ist die Anhaftung für das Überleben der Zellen und deren Vermehrung notwendig).

Nach den weiter unten beschriebenen Prozessschritten des Scannens und Detektierens der für den Nutzer interessanten Objekte erfolgt deren Positionierung zur Ernte. Der Werkzeugkopf nach DE 10 2004 046 740 wird zur Ernte vorbereitet, indem zunächst ein handelsüblicher Tip 12 aus Kunststoff aufgenommen wird und dieser Tip 12 mit einem für den jeweiligen Zelltyp optimierten Enzym oder Lösungsmittel (ggf. temperiert) gefüllt wird, um anschließend eine Klonierglocke 13 nach DE 197 42 163 C2 aufzunehmen. Mit dieser Klonierglocke 13 wird die zu vereinzelnde Kolonie umschlossen und durch Abgabe des Enzyms bzw. Lösungsmittels aus dem Tip 12 in den Innenraum der Klonierglocke 13 und damit auf das betreffende Objekt, entsprechende Spülzyklen und Einwirkzeiten und abschließendem Aufnehmen des Volumens innerhalb der Klonierglocke 13 in den Tip 12 wird die gewünschte Kolonie aus dem Probenbehälter vereinzelt und kann anschließend in andere Probenbehälter verbracht und dort weiter bearbeitet und untersucht werden.

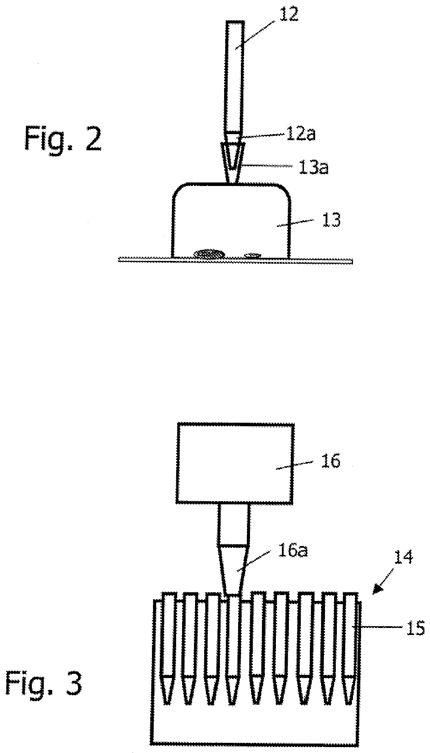

Die Fig. 2 zeigt hierzu ein erstes Entnahmewerkzeug 10a. Ein mit Flüssigkeit, insbesondere einem Lösungsmittel oder einem Enzym, füllbares Tip 12 ist mit einer Klonierglocke 13 kombiniert. Bei dem hier gezeigten Tip 12 handelt es sich um ein röhrenförmiges Gebilde in Form einer Pipette oder Kanüle, die einen Endkonus 12a aufweist, der in einen Aufnahmekonus 13a der Klonierglocke 13 eingesetzt wird und dort formschlüssig rastet. Zweckmäßigerweise wird das Tip 12 zunächst mit Flüssigkeit befüllt und nimmt außerhalb der Zellkultur 8 die Klonierglocke 13 auf. Der so gebildete Verbund aus Tip 12 und Klonierglocke 13 wird über den selektierten Zellen bzw. Zellkolonien in der Zellkultur 8 abgesetzt. Anschließend wird die Flüssigkeit innerhalb des Tips 12 in die Klonierglocke 13 abgegeben. Die dadurch abgelösten Zellen werden schließlich aus der Klonierglocke 13 in das Tip 12 abgesaugt. Ein derartiges Entnahmewerkzeug 10a eignet sich besonders für adhärente, d.h. am Boden eines Gefäßes anhaftende Zellen und Zellkolonien. Die Klonierglocke 13 erfasst dabei einen durch deren Radius festgelegten Bereich der Zellkultur 8. Der Radius der Klonierglocke 13 ist dabei in Abhängigkeit von der Dichte der Zellpopulation zu wählen. Ein besonderer Vorteil der Klonierglocke 13 besteht darin, dass die relative Positionierung zwischen Entnahmewerkzeug 10a und Zellkultur 8, die wie erwähnt über den xy-Tisch 9 ausgeführt wird, mit einer verhältnismäßig begrenzten Genauigkeit ausgeführt werden kann.

Zum Entfernen adhärenter Zellen und Zellkolonien, bei denen es erfahrungsgemäß unter Einwirkung von Enzymen oder Lösungsmitteln zu Beschädigungen an den Zellstrukturen kommt, kann auch auf eine mechanische Art des Ablösens der Zellen zurückgegriffen werden. Hierzu werden die selektierten Zellen bzw. Zellkolonien von der Spitze einer Kanüle 15 umschlossen und durch eine Relativbewegung von Kanüle 15 und Gefäß vom Untergrund durch ein Abkratzen gelöst. Die Kanüle 15 besteht dabei je nach Anwendungsfall oder Adhärenzstärke aus unterschiedlichen Materialien, beispielsweise Glas, Kunststoff oder Metall, und weist unterschiedliche Innendurchmesser auf, wobei mehrere Kanülen 15 in unterschiedlichen Ausführungen in einem Magazin bevorratet sein können.

Je nach den Erfordernissen an Sterilität und Durchsatz kann der Wechsel der Kanüle 15 manuell oder automatisch erfolgen. Für das automatische Wechseln der Kanüle 15 wird auf ein Kanülenmagazin 14 zurück gegriffen. Die Figur 3 zeigt ein Kanülenmagazin 14 mit einer Reihe von darin befindlichen Kanülen 15. Diese Anordnung kann als ein besonders dafür reservierter Teil des in Fig. 1 gezeigten Vereinzelungsmagazins 11 ausgebildet sein. Ein dafür vorgesehener Wechselkopf 16 weist einen Adapter 16a zum Erfassen und Herausziehen einer Kanüle 15 aus dem Kanülenmagazin 14 auf.

Ein Wechselkopf 16 wird über die Kanüle 15 bewegt und abgesenkt. Er ergreift die Kanüle 15 und bewegt diese über die Zellkultur. Dort erfolgt eine Verschiebung des xy-Tisches 9 an die Position der selektierten Zelle bzw. Zellkolonie. Die Kanüle 15 wird abgesenkt und umschließt die Zelle. Der xy-Tisch 9 führt nun langsame oszillierende Bewegungen aus, wobei in der Kanüle 15 dabei ein Unterdruck erzeugt wird, der die Zelle ansaugt.

Ernte stark adhärenter Zellkolonien (Stammzellen, Zellen auf Feederzellen usw.)durch mechanische Einwirkung:

Dieses Verfahren dient der vollständigen oder teilweisen Ablösung von Zellkolonien, welche stark am Boden von Probenbehältern anhaften (bei diesen Zelltypen ist die Anhaftung für das Überleben der Zellen und deren Vermehrung notwendig).

Der Werkzeugkopf 10 nach DE 10 2004 046 740, unterscheidet sich von dem zuvor erläuterten Werkzeugkopf 10 durch die Verwendung einer Kanüle 15 als Entnahmewerkzeug 10a. Die Kanüle 15 kann, je nach Anwendungsfall, aus unterschiedlichen Materialien (Kunststoff, Glas, Metall) bestehen und je nach Größe der zu erntenden Kolonien unterschiedliche Innen- und Außendurchmesser aufweisen. Der Wechsel der Kanüle 15 kann, je nach Erfordernissen an Sterilität und Durchsatz, manuell (meist kombiniert mit einem Desinfektionsschritt zwischen den Ernteprozessen) oder automatisch (spezielle Kanülen 15 werden ähnlich den Tips 17 in Racks vorgelegt) erfolgen.

Nach den weiter unten beschriebenen Prozessschritten des Scannens und Detektierens der für den Nutzer interessanten Objekte, erfolgt deren Positionierung zur Ernte. Mit der Kanüle 15 wird die zu vereinzelnde Kolonie umschlossen (Kanülenende liegt auf dem Boden des Probengefäßes auf). Das Ablösen der stark adhärenten Kolonie erfolgt mechanisch, durch Relativbewegungen der Kanüle 15 (Kratzen und damit Verschieben der eingeschlossenen Kolonie auf dem Boden des Gefäßes), ggf. auch in Kombination mit Saug- und Spülvorgängen der Spritze.

Die Relativbewegung wird durch Verfahren des xy-Tisches 9, des Entnahmewerkzeuges 10a oder beides in Kombination erzeugt. Auch die zusätzliche Anwendung von zelllösenden Enzymen innerhalb der Kanüle 15 kommt in Frage.

Nach Ablösen der Kolonie wird diese in der Kanüle 15 aufgenommen und in einen anderen Behälter verbracht. Die Kanüle 15 wird nun je nach Anwendung desinfiziert oder eine neue Kanüle 15 aufgenommen. Anschließend kann die nächste Kolonie geerntet werden.

Dieser Werkzeugkopf 16 mit Kanülen 15 entstand, nachdem mit dem zuvor genannten Werkzeugkopf 10 mit Tip 12 zur Ernte adhärenter Zellen mittels Enzym bei verschiedenen Zelltypen Probleme bei der Ablösung und der dafür benötigten Zeit auftraten. Die Enzyme zum Ablösen der Zellen greifen die Zellmembran an. Eine zu hohe Dosierung oder zu lange Einwirkzeit des Enzyms, wie sie für stark adhärente Zellen notwendig ist, führt oft zu deren Schädigung oder Vernichtung. Die Hauptanwendungen der Vorrichtung liegen aber in der Vereinzelung lebender Zellen, welche nach der Ernte weiter kultiviert und vermehrt werden sollen. Somit wurde für diese Zelltypen ein neues, automatisierbares Verfahren benötigt, um auch diese stark adhärenten Zellen vereinzeln zu können.

Durch die Verwendung von Kanülen 15 unterschiedlicher Durchmesser und Materialien mit planen Enden und einem konischen Adapter nach DE 10 2004 046 740, welcher die Aufnahme, Abgabe und Magazinierung der Kanülen 15 ermöglicht, und unter Zuhilfenahme entsprechender Vorrichtungen mit automatischen Abläufen sowie Werkzeugköpfen 10 zur Aufnahme der Kanülen 15 nach DE 10 2004 046 740 konnten erfolgreich Ernten an stark adhärenten Zelltypen ausgeführt werden.

Neben der Verwendung der Kanülen 15 ist die Verwendung von Relativbewegungen zum schonenden Ablösen der Einzelzellen und/oder Kolonien ein weiteres Merkmal der Erfindung. Diese Relativbewegung wird entweder durch die Kanüle 15 (Bewegung des Entnahmewerkzeuges), die Probe (Bewegung des xy-Tisches 9) oder durch beide ausgeführt. Richtung, Verfahrweg und Geschwindigkeit richten sich nach dem jeweiligen Zelltyp.

Die Auswahl der Durchmesser der Kanülen 15 erfolgt anhand der Größe der zu separierenden Zellen und/oder Kolonien. Durch die Verwendung der konischen Aufnahme 32 als Adapter (siehe Patentanmeldung DE 10 2004 046 740) können diese stark variierenden Kanülengrößen und Materialien mit dem gleichen Werkzeug gehandhabt werden. Spezielle Kanülen 15 - vor allem kleinerer Durchmesser oder nichtmetallischer Materialien - können in entsprechende konische Adapter eingeklebt werden.

Nach dem Ablegen der Zelle in dem Vereinzelungsmagazin 11 und einem optionalen Desinfektionsvorgang kann nunmehr die Kanüle 15 im Kanülenmagazin 14 abgelegt und eine neue Kanüle 15 entnommen werden.

Im Folgenden ist das Verfahren in Bezug auf Feederzellen beschrieben:
Die Ernte von Kolonien von Feederzellen mit Aufnahme von Feederzellen erfolgt mittels eines Scrape-Moduls. Die Kolonie wird durch eine Metallkapillare des Scrape-Moduls vollständig umschlossen oder ein Teil der Kolonie wird durch die Metallkapillare ausgestanzt. Dazu wird die Metallkapillare bei der Ernte auf den Boden der Kulturschale aufgesetzt. Die Kolonie umgebende Feederzellen oder unter der Kolonie liegende Feederzellen werden durch eine Scrape-Bewegung mit abgelöst und aufgenommen. Sie werden mit in das Zielwell abgelegt. Dies ist normalerweise nicht störend, da die Feederzellen sich nicht mehr teilen und nach einiger Zeit absterben.

Die Ernte von Kolonien von Feederzellen ohne Aufnahme von Feederzellen erfolgt mit der Glaskapillare. Dazu wird im Abstand von 0 - 50 $\mu$m über der Zielkolonie der obere Bereich der Kolonie mittels Einsaugen aufgenommen. Da die Kolonie ein dreidimensionales Objekt ist, wirken die Ansaugkräfte nur auf den oberen, dem Schalenboden abgewandten Bereich der Kolonie und nicht auf die Randbereiche oder auf Bereiche außerhalb der Kolonie. Die Größe und Tiefe des aufzunehmenden Stückes werden dabei durch den Durchmesser der Kapillare, den Abstand zur Kolonie, die Ansaugmenge und die Ansauggeschwindigkeit festgelegt und müssen für jeden Zelltyp empirisch ermittelt werden. Es werden dadurch nur Zellen oder ein Teil der Kolonie geerntet, ohne die umgebenden oder darunter liegenden Feederzellen. Es ist weiterhin möglich durch wiederholte Aufnahme an der gleichen Stelle mehrere klonale (genetisch identische) Stücke einer Kolonie zu ernten. Dazu muss der Abstand der Kapillarspitze zur Kolonie bei jeder nachfolgenden Ernte jedes Mal neu eingestellt werden, um immer die gleiche Ansaugkraft zu erzeugen.

Ernte von Kolonien aus halbfesten Nährböden (Agar, Methylzellulose) :

Dieses Verfahren dient der vollständigen oder teilweisen Entnahme von Zellkolonien, welche sich am Boden oder innerhalb der Nährmedien befinden.

Nach den weiter unten beschriebenen Prozessschritten des Scannens und Detektierens der für den Nutzer interessanten Objekte, erfolgt deren Positionierung zur Ernte. Der Werkzeugkopf 10 nach DE 10 2004 046 740 wird zur Ernte vorbereitet, indem ein spezieller Tip 17 aus Kunststoff aufgenommen wird, welcher durch einen größeren Innendurchmesser an seiner Spitze im Verhältnis zu seinem Aufnahmevolumen gegenüber handelsüblichen Kunststofftips gekennzeichnet ist (wurde vorher gekürzt). Dieser Tip 17 wird über der zu vereinzelnden Kolonie positioniert oder die Kolonie von diesem eingeschlossen. Durch Absaugen des Nährmediums in der Nähe der Kolonie bzw. des eingeschlossenen Inhaltes des speziellen Tips 17 werden die Kolonien mit dem Nährmedium aufgenommen und können anschließend in andere Probenbehälter verbracht und weiter bearbeitet und untersucht werden.

Agar:

Bei der Ernte aus Agar reicht oftmals das Einstechen in die vom Agar umschlossene Kolonie, damit Partikel am Tip 17 des Entnahmewerkzeuges 10a anhaften und diese dann in der Zielwell, welche mit Nährmedium gefüllt ist, abgespült werden. Ein spezielles Werkzeug für diese Anwendung benötigt dann keinen Spritzenantrieb zum Einsaugen der Partikel.

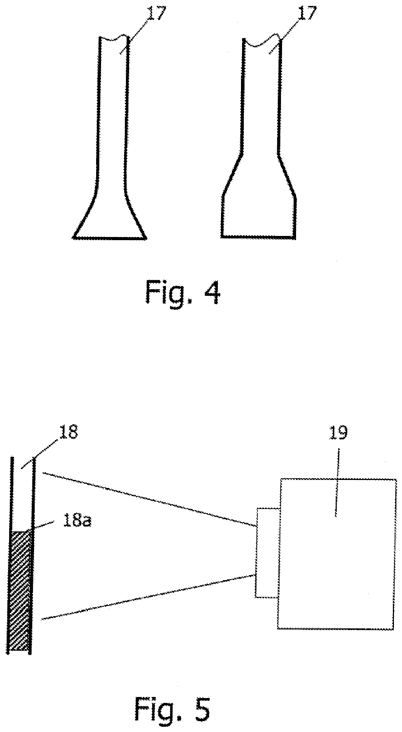

Die Fig. 4 zeigt zwei beispielhafte Tips 17 mit Spitzen mit einem erweiterten Innendurchmesser zum Entnehmen von Zellen aus halbfesten Nährboden, insbesondere Agar oder Methylzellulose. Das Tip 17 besteht zweckmäßigerweise aus Glas oder Kunststoff. Es wird über der zuvor selektierten Zelle positioniert und abgesenkt, wobei die Zelle oder Zellkolonie in der Spitze des Tips eingeschlossen wird. Über die aufgeweitete Spitze wird nun das Nährmedium mitsamt der enthaltenen Zelle bzw. Zellkolonie aufgenommen und kann in das Vereinzelungsmagazin verbracht werden.

Ernte von ortsfesten Einzelzellen:

Dieses Verfahren dient der Entnahme einzelner Zellen bzw. kleiner Zellkolonien, welche sich am Boden des Probenbehälters befinden und dort weitgehend ortsfest verbleiben, aber keine bzw. nur minimale Adhärenz aufweisen.

Nach den weiter unten beschriebenen Prozessschritten des Scannens und Detektierens der für den Nutzer interessanten Objekte erfolgt deren Positionierung zur Ernte. Der Werkzeugkopf 10 nach DE 10 2004 046 740 nimmt für diese Anwendung allerdings keinen Kunststofftip, sondern eine Kapillare auf. Diese wird, über den angeschlossenen Spritzenantrieb, je nach Zelltyp und applikativen Erfordernissen mit Luft oder einem Fluid gefüllt, mittels Bildverarbeitung kalibriert und so für den Ernteprozeß vorbereitet.

Die Kapillaröffnung (unterschiedliche Durchmesser je nach Zell- oder Koloniegröße) wird über der zu vereinzelnden Zelle oder Kolonie positioniert. Durch Absaugen des Nährmediums oder Puffers in der Nähe der Einzelzelle oder Kolonie wird die gewünschte Zelle oder Kolonie mit dem Medium aufgenommen und kann anschließend in andere Probenbehälter verbracht und weiter bearbeitet und untersucht werden.

Fig. 5 zeigt eine Kanüle 18 zur Entnahme von ortsfesten Einzelzellen. Eine derartige Kanüle 18 eignet sich zur Entnahme einzelner Zellen oder Zellkolonien, die sich am Boden eines Probenbehälters befinden und dort ortsfest, aber nicht adhärent verbleiben. Diese wird mit Luft oder einen Fluid befüllt, wobei der Fluidspiegel 18a der Kanüle 18 durch eine Bilderfassung 19 aufgenommen und kalibriert wird. Zur Entnahme der Zelle bzw. der Zellkolonie wird die Öffnung der Kanüle 18 über der Zelle bzw. Zellkolonie positioniert. Durch ein Absaugen des Nährmediums oder Puffers über der Zelle gelangt diese mit dem Medium in den Innenraum der Kanüle 18 und kann anschließend verbracht werden. Der Durchmesser der Kanülenöffnung muss dabei den Größen der Zellen angepasst sein.

Diese und zukünftig weitere Verfahren lassen sich durch Ergänzung der Basisplattform der Vorrichtung und deren Achssystem nach DE 10 2004 027 661 und der Verwendung einer vollständigen Mikroskopoptik realisieren.

Im Folgenden sollen der Prozess der Zelldetektion und der Bildverarbeitung näher erläutert werden.

Zuerst bestückt der Benutzer sowohl die Vorlagenaufnahme mit entsprechenden Zielplatten, Verbrauchsmaterialien und Flüssigkeiten sowie den Mikroskopkreuztisch mit seiner Ausgangsplatte, in welcher sich die zu erntenden Zellkulturen befinden. Diese Platten lassen sich in der Bildverarbeitungssoftware frei definieren und kalibrieren. Daraufhin können diese Platten abgescannt werden.

Zum Abscannen wird der Tisch in einem Raster verfahren, welches dem Bildausschnitt des optischen Kamerasystems entspricht. Dadurch kann Bild für Bild der Inhalt der kompletten Platte gescannt werden. Nachdem eines dieser Einzelbilder gescannt wurde erfolgt sofort eine auf Grauschwellwerten (und damit auf Helligkeitsunterschieden) basierende Detektion der Partikel. Entsprechende mathematische Filter können vor dieser Erkennung angewendet werden, um z.B. Kontraste zu optimieren oder das Bild für eine bessere Erkennung aufzubereiten. Diese Erkennung erfolgt Bild für Bild, also während dem Abscannen. Randübergreifende Partikel werden dabei von der Software automatisch erkannt und zu einem Partikel zusammengesetzt. Diese Art der Detektion wird daher auch als randübergreifende Detektion bezeichnet. Im Ergebnis bleibt in erster Linie nur eine sog. Partikelkarte übrig, welche in binärer Form zeigt, wo sich erkannte Partikel befinden und wo nicht. Es müssen also nicht aufwändig Bilddaten im Speicher gehalten werden sondern nur ein Abbild des Detektionsergebnis. Optional kann ein größenreduziertes Übersichtsbild erzeugt und abgespeichert werden. Zusätzlich zu den bereits erwähnten Filtern zur Bildaufbereitung können nach der Detektion noch weitere Filter angewendet werden. So können die erkannten Partikel in Hinsicht auf ihre morphologischen (Form, Größe usw.) und qualitativen Parameter (Dichte, Helligkeitsunterschiede usw.) bewertet und ausgefiltert werden. Dieses vorgehen birgt den Vorteil in sich, dass die Analyse der Partikel anhand der mit 100% Auflösung aufgenommenen Einzelbilder erfolgt, nicht mit einem evtl. qualitätsreduziertem Übersichtsbild.

All die verbliebenen Partikel werden in einer Partikelliste ausgegeben und können einzeln vom Benutzer angefahren, bewertet und nachbearbeitet werden.

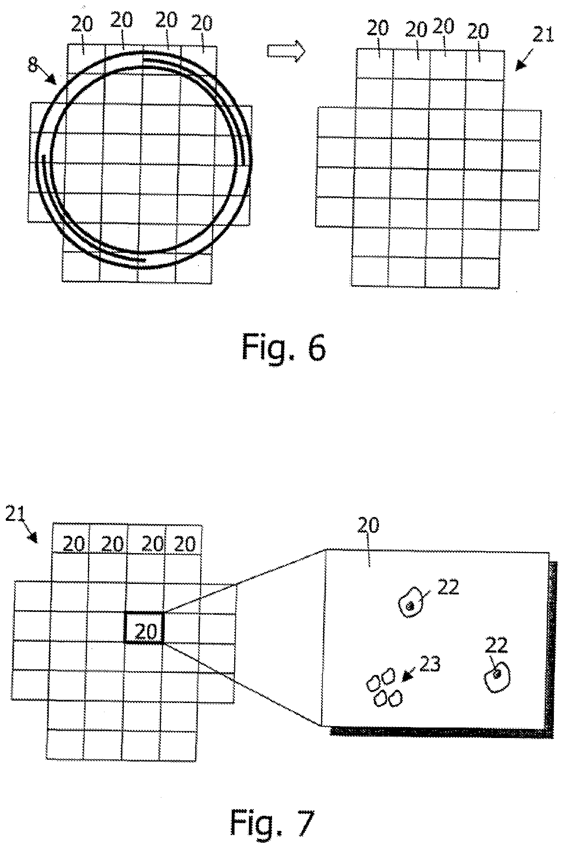

Die Fig. 6 zeigt im linken Teilbild schematisch die Zellkultur 8. Mittels einer Bewegung des xy-Tisches 9 wird die Zellkultur mit einer Reihe von mikroskopischen Einzelbildern 20 abgescannt. Die Größe der Einzelbilder hängt von dem an der Mikroskopeinheit 1 eingestellten Vergrößerungsfaktor ab. Je kleiner der Vergrößerungsfaktor ist, umso größer ist der durch ein Einzelbild 20 erfasste Ausschnitt der Zellkultur 8, umso weniger Einzelbilder 20 sind zur gesamten Aufnahme der Zellkultur 8 notwendig und umso größer sind die durch den xy-Tisch 9 auszuführenden Schrittbewegungen, um den nächst folgenden Bildausschnitt unter die Mikroskopeinheit zu führen.

Es ist demnach notwendig, die Schrittbewegungen des xy-Tisches 9 mit dem Vergrößerungsfaktor der Mikroskopeinheit 1 abzugleichen. Diese Abstimmung wird durch die Speicher- und Steuereinheit 4 bewirkt. Jedes der aufgenommenen Einzelbilder 20 ist dabei durch die Stellung des xy-Tisches 9 eindeutig in seinen und y-Koordinaten identifizierbar. Dabei lassen sich die innerhalb der Einzelbilder 20 gegebenen Koordinaten der dort enthaltenen Bildpunkte in einfacher Weise mit den Koordinaten des Einzelbildes verknüpfen. Dadurch gibt jeder Bildpunkt in jedem Einzelbild in eindeutiger Weise einen Ort in der gescannten Zellkultur 8 an.

Anhand der aufgenommenen Einzelbilder 20 der Zellkultur 8 kann der weiter unten beschriebene erste und/oder zweite Detektionsschritt ausgeführt werden, um die Zellen und/oder Zellkolonien nach bestimmten Parametern auszuwählen.

Alternativ dazu werden die so aufgenommenen Einzelbilder 20 in der Bildauswerteeinheit 3a zu einem Übersichtsbild 21 der gesamten Zellkultur 8 vereinigt. Diese Vereinigung ist zum einen deswegen sinnvoll, weil dadurch Strukturen, die an den Rändern der jeweiligen Einzelbilder 20 aufgenommen worden sind, zu vollständigen Objekten vervollständigt werden. Zum anderen erlaubt das Übersichtsbild 21 dem Benutzer einen schrittlos und kontinuierlich ausführbaren Überblick über die gescannte Zellkultur 8. Das Übersichtsbild 21 kann zu diesem Zweck durch eine Bildverarbeitungssoftware aufbereitet und dem Benutzer in verschiedenen Auflosungsstufen auf dem Monitor 4a angezeigt werden. In Verbindung mit der Generierung des Übersichtsbildes 21 erfolgt auch ein Abgleich der Koordinaten der Einzelbilder 20 und der Koordinaten der Bildpunkte innerhalb aneinander anschließender Einzelbilder, um Überdeckungen gleicher Bildbereiche möglichst auszuschließen.

Die Zelldetektion, d.h. die automatische Erkennung von Zellen oder Zellkolonien innerhalb der Einzelbilder 20 wird durch eine im Folgenden erläuterte Formerkennung ausgeführt. Die Fig. 7 zeigt hierzu ein aus dem Übersichtsbild 21 entnommenes beispielhaftes Einzelbild 20 mit darin enthaltenen Zellen 22 und einer Zellkolonie 23 im mikroskopischen Bild. Eine Voraussetzung für eine sichere Formerkennung der Zellen ist ein ausreichend großer Kontrast zwischen den Zellen bzw. Zellkolonien und deren Hintergrund im mikroskopischen Bild. Dies kann durch unterschiedliche Verfahren der Mikroskopietechnik erreicht werden. Eine erste Möglichkeit besteht darin, das optische System der Mikroskopeinheit 1 auf die Bildebene der Zellkultur 8 zu fokussieren, in der die Zellen zu erwarten sind. Bei adhärenten Zellen ist dies die Oberflache des Bodes des Probengefäßes der Zellkultur. Zellen in einem halbfesten Nährmedium, beispielsweise Agar, befinden sich in der Regel auf der Oberseite des Agars und können dort fokussiert werden.

Bei Zellen oder Zellkolonien, die sich innerhalb der Zellkultur in unterschiedlichen Bildebenen befinden können, lassen sich Techniken der Fluoreszenzmarkierungen mikobiologischer Objekte nutzen. Hierzu werden die zu identifizierenden Zellen mit einem Fluoreszenzmarker markiert, wahrend über die Beleuchtung Licht mit einer entsprechenden Anregungswellenlange eingestrahlt wird. Bei einer Bilderfassung im Bereich der Fluoreszenzwellenlange des Markers zeichnen sich die Zellen bzw. Zellkolonien vor einem dunklen Hintergrund als leuchtende bzw. helle Strukturen ab, die einen ausreichenden Farbkontrast bilden.

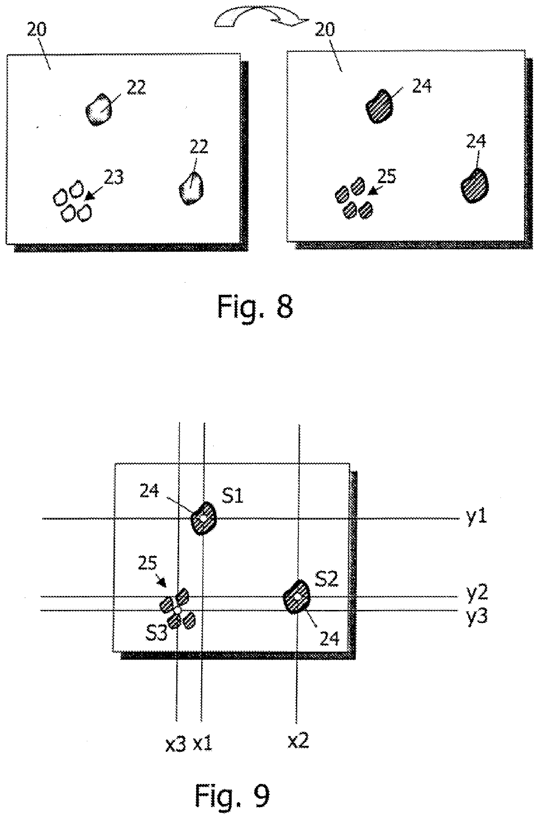

Die Fig. 8 zeigt einen weiteren schematischen Schritt der Bildverarbeitung. Im linken Bild ist ein in Grauwerte umgewandeltes Einzelbild 20 gezeigt. Die Umwandlung in Grauwerte ist besonders dann vorteilhaft, wenn sich die Zellen im mikroskopischen Bild hinreichend stark vom Hintergrund abheben. Natürlich ist auch eine Hervorhebung eines einzelnen Farbwertes des Bildes oder auch eine Reduktion des Bildes auf einen Farbwert möglich. Ebenso können die Farbwerte von Bildpunkten der zu detektierenden Zellen als Referenz vorgegeben sein, wobei jeder einzelne Bildpunkt innerhalb des Einzelbildes 20 mit diesem Referenzwert verglichen wird.

Bildpunkte, die vorgegebenen Referenzwerten, d.h. Farbwerten, Graustufen und dergleichen Werten, entsprechen, werden im Ablauf der Bildverarbeitung zu Punktmengen zusammengefasst, deren Form, Größe und Umriss analysiert werden können. Die Zellen 22 zeichnen sich zum Beispiel durch relativ große geschlossene Formen 24 aus, die einen bestimmten Bereich des Bildes kompakt einschließen, wobei deren Ränder im wesentlichen glatt verlaufen. Die Zellkolonie 23 hingegen bilden in einem Bildbereich eine Menge einzelner kleiner dicht beieinander liegender Strukturen 25 aus. Beide Formen können im Rahmen von gebräuchlichen Bilderkennnungsroutinen problemlos identifiziert werden.

Auf der Grundlage der erkannten Formen werden weitere Bildverarbeitungsschritte ausgeführt, bei der die Position der Formen und deren gegenseitige Abstände zu einander berechnet werden. Die Positionsbestimmung ist für die spätere Entnahme der Zellen bzw. Zellkolonie wichtig, die Bestimmung des Abstandes ist notwendig, um festzulegen, ob die gefundenen Zelle oder Zellkolonie durch einen einzelnen Entnahmevorgang oder zusammen mit anderen benachbarten Zellen der Zellkultur entnommen werden muss.

Ein Beispiel für die Positions- und Abstandsbestimmung ist in Fig. 9 dargestellt. Die Darstellung in Fig. 9 greift auf das in Fig. 8 schematisch gezeigte Ergebnis der Bildverarbeitung zurück. In dem hier gezeigten Beispiel wird von jeder Menge an Bildpunkten, die zu der erkannten Form, d.h. Zelle oder Zellkolonie, gehören, ein Formschwerpunkt S1, S2 bzw. S3 errechnet. Der Modus, nachdem diese Berechnung erfolgt, und welche Größe eines Bildausschnitts für dessen Berechnung zu wählen ist, kann durch den Benutzer vorab festgelegt werden. Dadurch lässt sich unter anderem definieren, ab wann eine Gruppe von gefundenen Zellen als eine Zellkolonie oder eine Gruppe von Einzelzellen behandelt werden soll.

In dem hier gezeigten Beispiel wird für die einzelnen Formen 24 jeweils ein eigener Formschwerpunkt S1 bzw. S2 errechnet, dem jeweils eine x-Koordinate x l bzw. x2 und eine y-Koordinate y l bzw. y2 zugeordnet wird. Für die dicht beieinander liegenden Formen 25 wird ein Formschwerpunkt S3 mit Koordinaten x3 und y3 berechnet, der für die gesamte Menge dieser Strukturen gilt und somit in einem Zwischenbereich dieser Formen liegt. Mit der Berechnung der Formschwerpunkte und der Angabe und Speicherung ihrer Koordinaten sind die Zellen bzw. Zellkolonien eindeutig in ihrer Lage identifiziert. Die Koordinaten werden zusammen mit den Bilddaten der Zellen und Zellkolonie in einer Positionsdatenbank gespeichert und lassen sich durch ein Aufrufen der Positionsdatenbank in eindeutiger Weise auffinden.

Zur Abstandsbestimmung der erkannten Formen und also der Zellen bzw. Zellkolonien wird auf die ermittelten Koordinaten zurückgegriffen, wobei die Abstände $|Sij|$ zwischen zwei beliebigen Formschwerpunkten $Si$ und $Sj$ und deren Koordinaten $(xi; yi)$ bzw. $(xj; yj)$ unter Verwendung der Pythagoras-Beziehung

$$|Sij| = \sqrt{(xi - xj)^2 + (yi - yj)^2}$$

errechnet werden. Diese Berechnung kann automatisch im Rahmen der Bildverarbeitung ausgeführt werden, wenn die ermittelten Zellen oder Zellkolonien innerhalb vorab festgelegter Grenzen nah beieinander aufzufinden sind. Außerdem besteht natürlich die Möglichkeit, dass durch den Benutzer im Rahmen einer Editierung der Positionsdatenbank eine manuelle Abstandsberechnung ausgeführt werden kann. Dabei wird softwareseitig auf Mittel für eine interaktive graphische Benutzerführung und Bildeditierung zurückgegriffen, bei der insbesondere einzelne Zellen per Mausklick markiert werden können und anschließend der Abstand zwischen den markierten Zellen durch das Bildverarbeitungsprogramm berechnet wird.

Die verwendete Standardbildverarbeitungssoftware verfügt über umfangreiche Möglichkeiten der weiteren Dokumentation der Detektionsbilder und Ergebnisse, wie grafische Auswertungen, Reportgenerator usw. Eine durchgängige Dokumentation des Prozesses ist damit möglich.

In einem zweiten Detektionsschritt ist es möglich, die bereits erkannten Partikel hinsichtlich weiterer Parameter zu untersuchen. Bei dieser Analyse wird nicht mehr die komplette Ausgangsplatte gescannt sondern nur noch die Bereiche, wo im ersten Analyseschritt interessantes Zellmaterial gefunden wurde. Dies optimiert die Ausführungszeit und im Falle einer Fluoreszenzanalyse auch die Beleuchtungsdauer der Probe mit Fluoreszenzlicht, welche minimal zu halten ist, um ein Ausbleichen der Probe zu verhindern. Der zweite Detektionsschritt kann aus beliebig vielen Einzelschritten bestehen, welche jeder mit anderen Belichtungsarten aufgenommen werden kann. So können viele verschiedene Fluoreszenzkanäle und Anregungswellenlängen bei dieser zweiten Detektion gescannt und bewertet werden. Die Software sammelt hierbei lediglich Partikeldaten, welche weniger Datenintensiv sind als Bilddaten.

Am Ende dieses zweiten Detektionsschritts werden alle gewonnenen Daten in die bereits vorhandenen Partikelliste zu den entsprechenden Originalpartikeln eingefügt. Dadurch ist ein entsprechender Überlagerungseffekt gegeben, womit die Daten aus der ersten Analyse mit den Daten der zweiten Analyse auf demselben Partikel miteinander verglichen werden können. So ergibt z.B. der Quotient aus Fluoreszenzfläche (Fläche zweite Analyse) geteilt durch die Fläche des Hellfeldpartikels (Fläche erste Analyse) ein Qualitätsmerkmal für die Antikörperproduktion (je heller und größer das Fluoreszenzsignal bei je kleinerer Hellfeldkolonie desto mehr produziert diese Kolonie Antikörper).

Diese Partikelliste kann weiterhin logisch über das Vorhandensein bzw. das Nicht-Vorhandensein einzelner Fluoreszenz- oder Hellfeldsignale gefiltert werden. Weiterhin hat der Nutzer die Möglichkeit, über ein 2-Dimensionales Scatter-Diagramm Partikel zu filtern. Hier werden zwei verschiedene Partikelwerte aus der Ergebnistabelle gegeneinander abgebildet.

Ist die Tabelle vollständig gefiltert, kann diese Liste entweder automatisch oder zeilengenau einzeln abgeerntet werden. Für die Durchführung des eigentlichen Ernteprozesses ist dabei die AVISO Robotersteuerungssoftware verantwortlich, die den Vorgang auf ein Signal der Bildverarbeitungssoftware hin startet. Die Bildverarbeitungssoftware übernimmt dabei die Positionierung des Kreuztisches und stellt sicher, dass sich das jeweils nächste zu erntende Objekt exakt zentriert im Sichtfeld der Kamera befindet, so dass das Erntewerkzeug, das vorher auf diese Position kalibriert wurde, die Zellen trifft und aufnehmen kann.

Die Kommunikation zwischen den beiden Softwarekomponenten erfolgt bidirektional. Dazu gehört, dass die Bildverarbeitungssoftware vor dem Beginn des Ernteprozesses Informationen wie Partikeldurchmesser oder Positionsindizes bei der Verwendung einer Multiwell-Platte an die Robotersteuerung sendet. Dadurch ist die Ablaufsteuerung in die Lage versetzt, den Erntevorgang flexibel für den jeweiligen Erntekandidaten durchzuführen. Eine typische Anwendung ist hierbei die Verwendung von automatisch wechselbaren Kanülen und/oder Kapillaren unterschiedlicher Form, Größe und Materials, um so den unterschiedlichen Größen der Partikel gerecht zu werden und optimale Ernteergebnisse zu erzielen. Ebenso ist es möglich, geerntete Zellen nach Klassen (Dichte, Fluoreszenz, ...) sortiert in die Abgabebehälter ablegen zu lassen, was für nachgelagerte Prozesse enorm wertvoll sein kann. Unabhängig davon erhält die Bildverarbeitungssoftware nach jedem Erntevorgang die Information, in welchen Behälter und an welcher Position in diesem Behälter die Robotersteuerung die Zelle(n) abgelegt hat, womit eine vollständige Protokollierung des Gesamtprozess gewährleistet ist.

Partikel, welche in ihrer Größe die verwendbaren Kanülendurchmesser überschreiten, können in Teilen nacheinander geerntet werden. Auch das Herauslösen nur von Teilen einer Kolonie (z.B. undifferenzierte Stammzellen, welche von differenzierenden umgeben sind) ist möglich.

Die jeweils zu erntende Kolonie, Teilkolonie oder Einzelzelle wird durch den Mikroskopkreuztisch in die optische Achse des Mikroskops positioniert. Es wird ein Bild des Partikels vor der Ernte aufgenommen und gespeichert. Gleiches erfolgt nach der Ernte, als Nachweis der erfolgreichen Ernte. Beide Bilder werden der entsprechenden Well des Zielgefäßes für diesen Partikel zugeordnet, so dass genau dieser Partikel in dieser Well für die weitere Untersuchung dokumentiert ist.

Die Koordinaten der Zellen bzw. Zellkolonien werden nunmehr zur Entnahme der Zellen genutzt. Hierzu wird das Erntemodul wie beschrieben über die Zellkultur 8 bewegt, wobei der xy-Tisch 9 jede Koordinate anfährt und das Erntemodul 5 der Werkzeugkopf 10 mit dem Entnahmewerkzeug 10a bei der entsprechenden Koordinate absenkt und die Zellentnahme aktiviert.

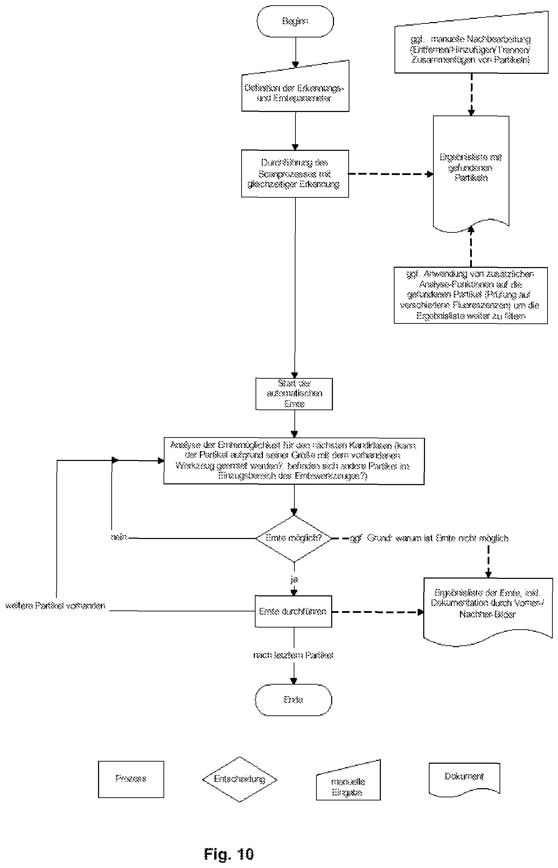

In Fig. 10 ist der grundsätzliche Ablauf der automatisierten Zellernte mit der Vorrichtung dargestellt. Zu Beginn des Verfahrens findet eine Definition der Erkennungs- und Ernteparameter statt. Anhand dieser Parameter findet dann die Durchführung des Scanprozesses mit gleichzeitiger Erkennung der Zellen und/oder Zellkolonien statt.

Die daraus ermittelten Daten werden in einer Ergebnisliste mit gefundenen Zellen und/oder Zellkolonien abgespeichert. Aus dieser Ergebnisliste können die gefundenen Zellen und/oder Zellkolonien weiter gefiltert werden. Dies erfolgt entweder durch eine manuelle Nachbearbeitung, d.h. Entfernen/Hinzufügen/Trennen/Zusammenfügen von Zellen und/oder Zellkolonien, oder durch eine Anwendung von zusätzlichen Analyse-Funktionen auf die gefundenen Zellen und/oder Zellkolonien. Zum Beispiel kann eine Prüfung auf verschiedene Fluoreszenzen erfolgen.

Nachdem die Zellen und/oder Zellkolonien auf diese Weise ausgewählt wurden, kann der Start der automatischen Ernte erfolgen. Dazu muss zunächst analysiert werden, ob die Zelle und/oder Zellkolonie aufgrund seiner Größe mit dem vorhandenen Entnahmewerkzeug geerntet werden kann und ob sich andere Zellen und/oder Zellkolonien im Einzugsbereich des Entnahmewerkzeugs befinden. Wenn die Ernte möglich ist, wird sie anschließend durchgeführt. Das Ergebnis der Ernte wird in der Ergebnisliste der Ernte inklusive einer Dokumentation der Vorher-/Nachher-Bilder abgespeichert. Danach erfolgt erneut die oben beschriebene Analyse der Erntemöglichkeit für die nächste Zelle und/oder Zellkolonie, wenn möglich mit anschließender Ernte.

Wenn die Ernte nicht möglich ist, wird zunächst der Grund dafür ermittelt und das Ergebnis wird in der Ergebnisliste der Ernte inklusive einer Dokumentation der Vorher-/Nachher-Bilder abgespeichert. Es folgt dann erneut die oben beschriebene Analyse für die nächste Zelle und/oder Zellkolonie mit anschließender Ernte, wenn möglich.

Nach der Ernte der letzten Zelle und/oder Zellkolonie wird das Verfahren automatisch beendet.

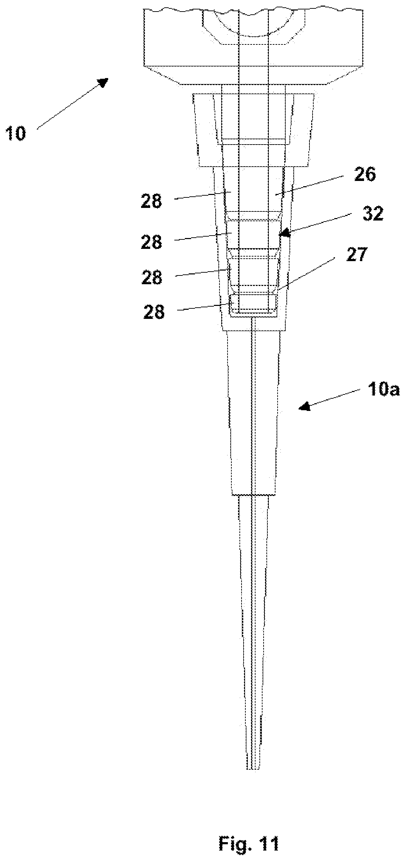

Fig. 11 zeigt eine Aufnahme 32, die am Werkzeugkopf 10 angebracht ist. Die Aufnahme 32 weist einen in Absätzen 28 abgestuften Außenkonus 26 auf. Dieser Außenkonus 26 nimmt ein Entnahmewerkzeug 10a auf, das hier ein Tip ist und einen Innenkonus 27 aufweist, der ein Gegenstück zum Außenkonus 26 der Aufnahme 32 bildet. Durch das Ineinandergreifen des Außenkonus 26 der Aufnahme 32 und des Innenkonus 27 des Entnahmewerkzeugs 10a entsteht eine kraftschlüssige, lösbare Verbindung zwischen der Aufnahme 32 und dem Entnahmewerkzeug 10a.

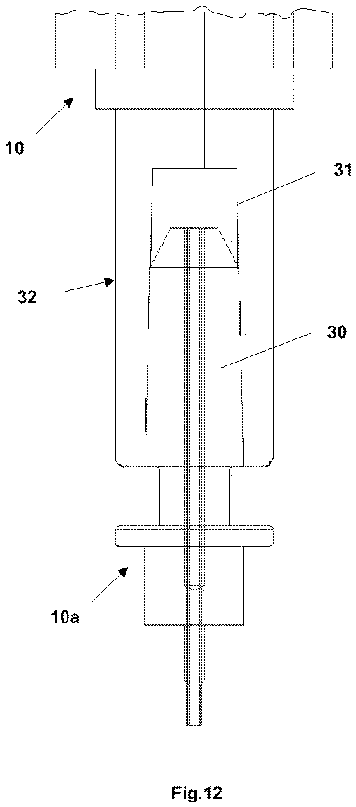

Fig. 12 zeigt eine alternative Ausführungsform einer Aufnahme 32. Die Aufnahme 32 weist hier einen Innenkonus 31 auf. Dieser Innenkonus 31 nimmt ein Entnahmewerkzeug 10a auf, das hier eine Kapillare ist und einen Außenkonus 30 aufweist, der ein Gegenstück zum Innenkonus 31 der Aufnahme 32 bildet. Durch das Ineinandergreifen des Innenkonus 31 der Aufnahme 32 und des Außenkonus 30 des Entnahmewerkzeugs 10a entsteht eine kraftschlüssige, lösbare Verbindung zwischen der Aufnahme 32 und dem Entnahmewerkzeug 10a.

Durch die Verwendung unterschiedlicher, in ihrer Größe und Ausführung abgestimmter konischer Aufnahmen 32 für die unterschiedlichsten verwendeten, oftmals neu entwickelten Verbrauchsmaterialien in Form von wechselbaren Kanülen, kann eine weitgehend einheitliche Werkzeuggeometrie beibehalten werden.

Die konische Aufnahme 32 führt zu einer Selbstzentrierung der teilweise hochpräzise zu positionierenden Verbrauchsmaterialien (Kanülen) bei der Aufnahme durch das Werkzeug.

Durch die hohe Stabilität der konischen Aufnahme 32 und der gleichmäßigen Kraftverteilung ist das Aufbringen relativ hoher Querkräfte auf die Wechselkapillaren z.B. beim Scrapen möglich, ohne dass der Verlust der Positionsgenauigkeit oder eine Lockerung der Wechselkanüle in der Aufnahme 32 auftritt.

Durch die Verwendung einer Verdickung unterhalb der konischen Aufnahme 32 am Werkzeugkopf, kann die Wechselkanüle mittels einer einfachen nicht dargestellten Abstreifvorrichtung wieder vom Werkzeug entfernt werden, um die Aufnahme der nächsten Wechselkanüle zu ermöglichen.

Dieser Bund dient gleichzeitig dazu, die Magazinierung auch empfindlicher Verbrauchsmaterialien, wie z.B. Glaskapillaren kleiner Durchmesser, zu ermöglichen, um diese in großer Stückzahl dem automatisierten Prozess zuzuführen. Hierzu werden in aller Regel 96er Racks verwendet.

Es ist die Verwendung verschiedener konischer Aufnahmen 32 zur Adaption unterschiedlichster automatisiert wechselbarer Kapillaren und Kanülen unterschiedlichster Größe, Form und Materialien an einem gleichartigen Werkzeug zum Zwecke der automatisierten Untersuchung und Separation von Einzelzellen oder Zellkolonien möglich.

Es können Kapillaren aus Glas, Metall oder Keramik verwendet werden.

Bezugszeichenliste

| | |
|---|---|
| 1 | Mikroskopeinheit |
| 1a | Umlenkprisma |
| 1b | Linsensystem |
| 2 | Bildaufnahmeeinheit |
| 3 | PC |
| 3a | Bildauswerteeinheit |
| 4 | Steuer- und Speichereinheit |
| 4a | Monitor, Display |
| 5 | Erntemodul |
| 5a | Hubsäule |
| 5b | Verfahrantrieb |
| 6 | Beleuchtung |
| 7 | Beleuchtungsfilter |
| 8 | Zellkultur |
| 9 | xy-Tisch |
| 10 | Werkzeugkopf |
| 10a | Entnahmewerkzeug |
| 11 | Vereinzelungsbatterie |
| 12 | Tip |
| 12a | Endkonus |
| 13 | Klonierglocke |
| 13a | Aufnahmekonus |
| 14 | Kanülenmagazin |
| 15 | Kanüle |
| 16 | Wechselkopf |
| 16a | Adapter |
| 17 | Tip |
| 18 | Kanüle für ortsfeste Einzelzelle |
| 18a | Fluidspiegel |
| 19 | Kalibrierende Bilderfassung |
| 20 | Einzelbild |
| 21 | Übersichtsbild |
| 22 | Zelle |
| 23 | Zellkolonie |
| 24 | Zellform |
| 25 | Zellkoloniestruktur |
| S1 | erster Formschwerpunkt |
| S2 | zweiter Formschwerpunkt |
| S3 | dritter Formschwerpunkt |
| x1 | erste x-Koordinate |
| x2 | zweite x-Koordinate |
| x3 | dritte x-Koordinate |
| y1 | erste y-Koordinate |
| Y2 | zweite y-Koordinate |
| y3 | dritte y-Koordinate |
| 26 | Außenkonus |
| 27 | Innenkonus |
| 28 | Absatz |
| 30 | Außenkonus |
| 31 | Innenkonus |
| 32 | Aufnahme |

**Patentansprüche**

1. Vorrichtung zur Entnahme von Zellen und/oder Zellkolonien aus einer Zellkultur, **gekennzeichnet durch** eine Mikroskopeinheit (1) zur mikroskopischen Abtastung der Zellkultur (8) in Kombination mit einer Bilderfassungseinheit (2) und einer Bildauswerteeinheit (3) zur Positionsdetektierung der Zellen und/oder der Zellkolonien in der Zellkultur (8), eine Steuer- und Speichereinheit (4) zum Speichern der detektierten Position der Zellen und/oder Zellkolonien und ein Erntemodul (5) mit einem Entnahmewerkzeug (10a) zur Entnahme der Zellen und/oder Zellkolonien an der detektierten Position der Zelle und/oder Zellkolonie, **dadurch gekennzeichnet, dass** die Vorrichtung einen Werkzeugkopf (10) mit einer Aufnahme (32) aufweist, die zur Herstellung einer kraftschlüssigen, lösbaren Verbindung zwischen dem Werkzeugkopf (10) und dem Entnahmewerkzeug (10a) aus einem Innenkonus (31) und/oder einem Außenkonus (26) gebildet ist und das Entnahmewerkzeug (10a) hält, das einen zur Aufnahme (32) passenden Innenkonus (27) und/oder Außenkonus (30) aufweist.

2. Vorrichtung nach Anspruch 1, **gekennzeichnet durch** ein Entnahmewerkzeug (10a) aus einem mit einer lösenden oder enzymatischen Flüssigkeit füllbaren Tip (12), einer an das Tip (12) ankoppelbaren, ausgewählte Zellen und/oder Zellkolonien überdeckenden, mit der innerhalb des Tips (12) enthaltenen Flüssigkeit füllbaren Klonierglocke (13) sowie einer Beschickungs- und Absaugevorrichtung für das Tip (12) und die Klonierglocke (13).

3. Vorrichtung nach Anspruch 1, **gekennzeichnet durch** ein Entnahmewerkzeug (10a) in Form eines Magazins (14) mit einer Anordnung aus innerhalb des Magazins (14) gelagerten Kanülen (15) unterschiedlichen Durchmessers und einer Koppeleinheit zur selbsttätigen Entnahme der Kanülen (15) aus dem Magazin (14) und deren Integration in einen Wechselkopf (16).

4. Vorrichtung nach Anspruch 3, **dadurch gekennzeichnet, dass** die Kanülen (15) in unterschiedlichen Größen, Formen und/oder Materialien ausgebildet sind.

5. Vorrichtung nach Anspruch 1, **gekennzeichnet durch** ein Entnahmewerkzeug (10a) mit einem Saugtip (17) mit einem vergrößerten Querschnitt in dessen Spitzenabschnitt.

6. Vorrichtung nach Anspruch 1, **gekennzeichnet durch** ein Entnahmewerkzeug (10a) mit einer Kanüle (18), einer Bildaufnahmevorrichtung (19) zum Überwachen einer in der Kanüle (18) enthaltenen Fluidmenge, einer Bildverarbeitungseinrichtung zum Verarbeiten der Bildinformation der Kanüle (18) und einer Saugvorrichtung zum Absaugen von Zellen und/oder Zellkolonien in die Kanüle (18).

7. Vorrichtung nach Anspruch 1, **dadurch gekennzeichnet, dass** der Innenkonus (31) und/oder der Außenkonus (26) der Aufnahme (32) aus Absätzen (28) bestehen, an denen das Entnahmewerkzeug (10a) gehalten ist.

8. Vorrichtung nach Anspruch 6, **dadurch gekennzeichnet, dass** das Entnahmewerkzeug (10a) eine Kanüle ist und dass der Werkzeugkopf (10) einen automatisierten Wechsel des Entnahmewerkzeuges (10a) zulässt.

9. Vorrichtung nach einem der vorstehenden Ansprüche, **dadurch gekennzeichnet, dass** das Entnahmewerkzeug (10a) eine automatisiert wechselbare Kapillare oder Kanüle (15) hält.

10. Vorrichtung nach einem der vorstehenden Ansprüche, **dadurch gekennzeichnet, dass** die konische Aufnahme (32) so ausgebildet ist, dass sie eine Selbstzentrierung einer Kanüle (15) bei der Aufnahme durch das Entnahmewerkzeug (10a) bewirkt.

11. Vorrichtung nach einem der vorstehenden Ansprüche, **dadurch gekennzeichnet, dass** sie eine Abstreifvorrichtung aufweist.

12. Vorrichtung nach einem der vorstehenden Ansprüche, **dadurch gekennzeichnet, dass** eine Verdickung unterhalb der konischen Aufnahme (32) am Werkzeugkopf (10) ausgebildet ist, wobei die Verdickung zum Entfernen einer Kanüle (15) oder Kapillare bestimmt ist.

13. Vorrichtung nach Anspruch 12, **dadurch gekennzeichnet, dass** eine Verdickung unterhalb der konischen Aufnahme (32) am Werkzeugkopf (10) ausgebildet ist, wobei die Verdickung zum Entfernen einer Kanüle (15) oder Kapillare mittels einer Abstreifvorrichtung bestimmt ist.

14. Vorrichtung nach einem der Ansprüche 3 bis 13, **dadurch gekennzeichnet, dass** die Verdickung eine Magazinierung von Kanülen (15) oder Kapillaren in dem Magazin (14) ermöglicht.

15. Vorrichtung nach einem der vorstehenden Ansprüche, **dadurch gekennzeichnet, dass** die konische Aufnahme (32) eine Stabilität und Kraftverteilung besitzt, die das Aufbringen hoher Querkräfte auf die Kanüle (15) oder Kapillare ermöglicht.

**Claims**

1. A device for the removal of cells and/or cell colonies from a cell culture, **characterized by** a microscope unit (1) for microscopic scanning the cell culture (8) in combination with an image acquisition unit (2) and an image evaluation unit (3) for the position detection of the cells and/or the cell colonies in the cell culture (8), a control and storage unit (4) for storing the detected position of the cells and/or the cell colonies and a harvesting module (5) with a removal tool (10a) for removing cells and/or the cell colonies at the detected position of the cell and/or the cell colony, **characterized in that** the device has a tool head (10) with a receptacle (32) formed to make a non-positive, detachable connection between the tool head (10) and the removal tool (10a) from an internal taper (31) and/or an external taper (26) and which holds the removal tool (10a) that has an internal taper (27) and/or external taper (30) that match with the receptacle (32).

2. The device according to claim 1, **characterized by** a removal tool (10a) consisting of a tip (12) that can be filled with a dissolving or enzymatic liquid, a cloning bell jar (13) that can be coupled to the tip (12), covers selected cells and/or cell colonies and can be filled with the liquid within the tip (12), as well as a feeding and suction device for the tip (12) and the cloning bell jar (13).

3. The device according to claim 1, **characterized by** a removal tool (10a) in the form of a magazine (14) with an assembly of cannulas (15) of different diameters stored within the magazine (14) and a coupling unit for the automatic removal of the cannulas (15) from the magazine (14) and their integration into an interchangeable head (16).

4. The device according to claim 3, **characterized in that** the cannulas (15) are formed in different sizes, shapes, and/or materials.

5. The device according to claim 1, **characterized by** a removal tool (10a) with a suction tip (17) having an enlarged cross section in its tip portion.

6. The device according to claim 1, **characterized by** a removal tool (10a) with a cannula (18), an image capturing device (19) for monitoring an amount of fluid contained in the cannula (18), an image processing facility for processing the image information of the cannula (18), and a suction device for suck off cells and/or cell colonies into the cannula (18).

7. The device according to claim 1, **characterized in that** the internal taper (31) and/or the external taper (26) of the receptacle (32) consist of steps (28) on which the removal tool (10a) is held.

8. The device according to claim 6, **characterized in that** the removal tool (10a) is a cannula and that the tool head (10) enables the automated exchange of the removal tool (10a).

9. The device according to any of the preceding claims, **characterized in that** the removal tool (10a) holds an automatically exchangeable capillary or cannula (15).

10. The device according to any of the preceding claims, **characterized in that** the tapered receptacle (32) is formed such that it causes a self-centering of a cannula (15) during pick up by the removal tool (10a).

11. The device according to any of the preceding claims, **characterized in that** it has a stripping device.

12. The device according to any of the preceding claims, **characterized in that** a thickening is formed below the tapered receptacle (32) on the tool head (10), wherein the thickening is for removing a cannula (15) or capillary.

13. The device according to claim 12, **characterized in that** a thickening is formed below the tapered receptacle (32) on the tool head (10), wherein the thickening is for removing a cannula (15) or capillary by means of a stripping device.

14. The device according to any of claims 3 bis 13, **characterized in that** the thickening enables storing of cannulas (15) or capillaries in the magazine (14).

15. The device according to any of the preceding claims, **characterized in that** the tapered receptacle (32) has a stability and force distribution which enable application of high shear forces to the cannula (15) or capillary.

**Revendications**

1. Dispositif pour prélever des cellules et/ou des colonies de cellules dans une culture cellulaire, **caractérisé par** une unité de microscope (1) pour l'exploration microscopique de la culture cellulaire (8) en combinaison avec une unité de visualisation d'images (2) et une unité de restitution d'images (3) pour la détection de positionnement des cellules et/ou des colonies de cellules dans la culture cellulaire (8), une unité de commande et de mémoire (4) pour mémoriser la position détectée des cellules et/ou des colonies de cellules et un module de récolte (5) avec un outil de prélèvement (10a) pour prélever les cellules et/ou les colonies de cellules à la position détectée de la cellule et/ou de la colonie de cellules, **caractérisé par le fait que** le dispositif présente une tête d'outil (10) avec accouplement (32) qui est formé d'un cône intérieur (31) et/ou d'un cône extérieur (26) et maintient l'outil de captage pour permettre une liaison détachable par adhérence entre la tête d'outil (10) et le outil de prélèvement (10a), ce dernier présentant un cône intérieur (27) et/ou un cône extérieur (30) adapté à l'accouplement (32).

2. Dispositif selon la revendication 1, **caractérisé par** un outil de prélèvement (10a) formé d'une pointe de pipette (12) remplissable d'un liquide solvant ou enzymatique, d'une cloche de clonage (13) remplissable avec le liquide contenu dans la pipette (12) et accouplée à la pipette (12) recouvrant les cellules sélectionnées et/ou les colonies sélectionnées de cellules, ainsi qu'un dispositif de déposition et d'aspiration pour la pipette (12) et la cloche de clonage (13).

**3.** Dispositif selon la revendication 1, **caractérisé par** un outil de prélèvement (10a) formé par un magasin (14) avec une ensemble de canules (15) de différents diamètres entreposées hors du magasin (14) et par une unité de couplage pour le prélèvement automatique des canules (15) dans le magasin (14) et leur intégration dans une tête interchangeable (16).

**4.** Dispositif selon la revendication 3, **caractérisé par le fait que** les canules (15) sont de grandeurs, de formes et de matériaux différents.

**5.** Dispositif selon la revendication 1, **caractérisé par** un outil de prélèvement (10a) avec une pointe de pipette d'aspiration (17) de section plus grande dans sa partie en pointe.

**6.** Dispositif selon la revendication 1, **caractérisé par** un outil de prélèvement (10a) avec une canule (18), un dispositif de prise d'images (19) pour la surveillance d'une quantité de fluide contenu dans la canule (18), par un appareillage de traitement d'images pour traiter l'information d'image de la canule (18) et par un dispositif d'aspiration pour transporter les cellules et/ou les colonies de cellules dans la canule (18).

**7.** Dispositif selon la revendication 1, **caractérisé par le fait que** le cône intérieur (31) et/ou le cône extérieur (26) d'accouplement (32) forment des épaulements (28) sur lesquelles l'outil de prélèvement (10a) est retenu.

**8.** Dispositif selon la revendication 6, **caractérisé par le fait que** l'outil de prélèvement (10a) est une canule et que la tête d'outil (10) permet un changement automatique de l'outil de prélèvement (10a).

**9.** Dispositif selon une des revendications précédentes, **caractérisé par le fait que** l'outil de prélèvement (10a) porte un capillaire ou une canule (15) automatiquement interchangeable.

**10.** Dispositif selon une des revendications précédentes, **caractérisé par le fait que** l'accouplement conique (32) est formé de telle sorte qu'il assure le centrage automatique d'une canule (15) lors de l'accouplement par l'outil de prélèvement (10a).

**11.** Dispositif selon une des revendications précédentes, **caractérisé par le fait qu'**il présente un dispositif de raclage.

**12.** Dispositif selon une des revendications précédentes, **caractérisé par le fait qu'**un épaississement est formé sous l'accouplement conique (32) sur la tête d'outil (10), ledit épaississement étant prévu pour l'enlèvement d'une canule (15) ou d'un capillaire.

**13.** Dispositif selon la revendication 12, **caractérisé par le fait qu'**un épaississement est formé sous l'accouplement conique (32) sur la tête d'outil (10), ledit épaississement étant prévu pour l'enlèvement d'une canule (15) ou d'un capillaire au moyen d'un dispositif de raclage.

**14.** Dispositif selon une des revendications 3 à 13, **caractérisé par le fait que** l'épaississement permet un emmagasinement de canules (15) ou de capillaires dans le magasin (14).

**15.** Dispositif selon une des revendications précédentes, **caractérisé par le fait que** l'accouplement (32) possède une stabilité et une répartition des forces qui permettent d'apporter de plus importantes forces transversales sur la canule (15) ou le capillaire.

Fig. 1

Fig. 2

Fig. 3

Fig. 4

Fig. 5

Fig. 6

Fig. 7

Fig. 8

Fig. 9

Begin

ggf. manuelle Nachbearbeitung
(Entfernen/Hinzufügen/Trennen/
Zusammenfügen von Partikeln)

Definition der Erkennungs-
und Erntepararameter

Durchführung des
Scanprozesses mit
gleichzeitiger Erkennung

Ergebnisliste mit
gefundenen
Partikeln

ggf. Anwendung von zusätzlichen
Analyse-Funktionen auf die
gefundenen Partikel (Prüfung auf
verschiedene Fluoreszenzen) um die
Ergebnisliste weiter zu filtern

Start der
automatischen
Ernte

Analyse der Erntemöglichkeit für den nächsten Kandidaten (kann
der Partikel aufgrund seiner Größe mit dem vorhandenen
Werkzeug geerntet werden? befinden sich andere Partikel im
Einzugsbereich des Erntewerkzeuges?)

Ernte möglich?        ggf. Grund warum ist Ernte nicht möglich

nein

ja

weitere Partikel vorhanden        Ernte durchführen

Ergebnisliste der Ernte, inkl.
Dokumentation durch Vorher-/
Nachher-Bilder

nach letztem Partikel

Ende

Prozess        Entscheidung        manuelle
Eingabe        Dokument

**Fig. 10**

23

10

28

28

28

28

26

32

27

10a

Fig. 11

**10**

**31**

**32**

**30**

**10a**

Fig.12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19742163 C2
- DE 102004027661 A1
- DE 102004046740 A1
- DE 19742163 A1
- EP 1502649 A1

- EP 1754537 A1
- DE 102004027661
- DE 102004046740